# FASCICULE DE BREVET EUROPEEN

(11) **EP 3 024 859 B1**
(45) Date de publication et mention de la délivrance du brevet: **27.03.2019**
(21) Numéro de dépôt: 14748247.5
(22) Date de dépôt: 18.07.2014
(51) Int. Cl.: C08F 220/58, A61K 8/81, A61Q 19/00

(54) **NOUVEAU POLYMÈRE EN POUDRE, PROCÉDÉ POUR SA PRÉPARATION ET UTILISATION COMME ÉPAISSISSANT**
NEUARTIGES PULVERPOLYMER, VERFAHREN ZUR HERSTELLUNG DAVON UND VERWENDUNG ALS EIN VERDICKUNGSMITTEL
NOVEL POWDER POLYMER, METHOD FOR THE PREPARATION THEREOF, AND USE AS A THICKENER

(30) Priorité: 24.07.2013 FR 1357280
(43) Date de publication de la demande: 01.06.2016
(73) Titulaire: Société d'Exploitation de Produits pour les Industries Chimiques SEPPIC, 75321 Paris cedex 07 (FR)
(72) Inventeur: BRAUN, Olivier, F-42330 Saint Bonnet les Oules (FR); MALLO, Paul, F-78110 Le Vésinet (FR)
(74) Mandataire: Laigneau, Amandine
(86) Numéro de dépôt international: PCT/FR2014/051851
(87) Numéro de publication internationale: WO 2015/011380

(56) Documents cités:
- EP-A2- 0 503 853
- EP-A2- 0 750 899
- WO-A1-2004/063228
- US-A1- 2007 004 851
- US-A1- 2011 098 364
- US-B1- 6 197 287

## Description

L'invention a pour objet de nouveaux agents épaississants ainsi que leur utilisation en cosmétique et en pharmacie.

L'industrie cosmétique et l'industrie pharmaceutique utilisent très régulièrement des polymères épaississants synthétiques pour augmenter la viscosité des crèmes, des émulsions et de diverses solutions topiques. Les polymères épaississants synthétiques actuellement utilisés en ces domaines, se présentent sous deux formes physiques, la forme poudre et la forme liquide pour laquelle le polymère est préparé par polymérisation en émulsion inverse à l'aide d'agents tensioactifs et que l'on appelle couramment latex inverse.

Les polymères épaississants sous forme de poudre, les plus connus sont les polymères à base d'acide acrylique ou les copolymères à base d'acide acrylique et de ses esters. On citera par exemple, les polymères commercialisés sous le nom de marque, CARBOPOL™ et PEMULEN™ et décrits notamment dans les brevets américains 5,373,044 et 2,798,053 ainsi que dans le brevet européen EP 0 301 532.

En cosmétique, on utilise aussi, et toujours sous forme de poudre, des homopolymères ou copolymères à base d'acide 2-acrylamido-2-méthyl-propane sulfonique. Ces polymères épaississants sont commercialisés sous le nom de marque ARISTOFLEX™ et décrits dans les demandes de brevet européen publiées sous les numéros EP 0 816 403, EP 1 116 733 et EP 1 069 142. Ces épaississants sous forme de poudre sont obtenus par polymérisation précipitante ; le (ou les) monomère(s) est (ou sont) mis en solution dans un solvant organique type benzène, acétate d'éthyle, cyclohexane, tertio-butanol. Ce procédé nécessite donc de nombreuses étapes successives de purification du produit final, pour éliminer toute trace de solvant résiduel.

L'industrie cosmétique utilise aussi très largement des épaississants sous forme de latex inverses et notamment ceux commercialisés par la demanderesse. On citera par exemple, les épaississants SEPIGEL™ 305, SIMULGEL™ 600, SIMULGEL™ EG, SIMULGEL™ EPG, SIMULGEL™ NS, SIMULGEL™ A, SEPIPLUS™ 400, SEPIPLUS™ 250 et SEPIPLUS™ 265. Ces épaississants sont obtenus par polymérisation en émulsion inverse. Ils présentent l'avantage d'être plus aisément manipulables et se dispersent très rapidement dans l'eau. De plus, ces produits développent des performances épaississantes remarquablement élevées ; ces performances sont probablement la conséquence du procédé pour leur préparation, une réaction de polymérisation en phase dispersée, qui conduit à des polymères de très hauts poids moléculaires.

Néanmoins, ces épaississants sous forme de latex inverse contiennent une huile et un ou plusieurs agents tensioactifs qui peuvent parfois induire des réactions d'intolérance cutanée sur des sujets particulièrement sensibles ; de plus cette présence d'huile les rend inutilisables pour la préparation de gels clairs.

Les inventeurs ont alors développé des épaississants ayant les performances épaississantes équivalentes ou supérieures aux latex inverses, mais mieux tolérés par la peau, en particulier du fait de l'absence de toute phase huile pouvant conduire à des gels plus clairs. Ces produits se présentent sous forme de poudre mais possèdent des temps de dissolution, et donc une facilité de mise en oeuvre, comparable à ceux des produits liquides. Ces composés, décrits dans la demande de brevet européen publiée sous le numéro EP 1 496 081, sont obtenus par les techniques classiques de polymérisation, qu'il s'agisse d'une polymérisation en phase dispersée, d'une polymérisation en suspension inverse, d'une polymérisation en émulsion inverse ou en micro-émulsion inverse ; les systèmes épaississants obtenus sont ensuite extraits et purifiés par différentes techniques telles que la précipitation dans un tiers solvant, la précipitation dans un tiers solvant suivie éventuellement d'un lavage, d'un séchage par atomisation ou par déshydratation azéotropique, suivie éventuellement d'un lavage par un solvant judicieusement choisi. Ces épaississants combinent donc une partie des avantages des épaississants en poudre classique (absence d'huile, obtention de gels plus clairs) et les avantages des épaississants sous forme de latex inverse (haute vitesse de dissolution dans l'huile pouvoir épaississant et stabilisant remarquable). Cependant pour certaines utilisations, les clients utilisateurs de tels systèmes épaississants souhaitent pouvoir fabriquer des gels encore plus clairs, que ceux obtenus aujourd'hui, voir des gels transparents. De plus, les gels obtenus avec ces épaississants n'ont pas une stabilité satisfaisante lorsque la composition est riche en électrolytes comme c'est souvent le cas des compositions comprenant des filtres solaires.

Les inventeurs ont ensuite cherché à développer de nouveaux systèmes épaississants qui n'aient pas les inconvénients cités ci-dessus et qui présentent des propriétés épaississantes suffisantes pour qu'ils soient une alternative acceptable aux compositions de l'état de la technique. C'est l'objet de la demande de brevet français publiée sous le numéro 2 879 607 qui a notamment pour objet, un polyélectrolyte anionique, terpolymère linéaire, branché ou réticulé d'au moins un monomère possédant une fonction acide fort, libre, partiellement salifiée ou totalement salifiée, avec au moins un monomère neutre, et au moins un monomère de formule (I) : 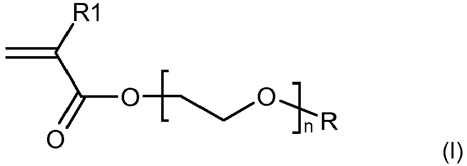 dans laquelle R1 représente un atome d'hydrogène ou un radical méthyle, R représente un radical alkyle linéaire ou ramifié comportant de huit à trente atomes de carbone et n représente un nombre supérieur ou égal à un et inférieur ou égal à cinquante. Ces polymères présentent des propriétés épaississantes, notamment en présence d'électrolytes, très importantes. En particulier il faut noter que les capacités épaississantes sont très peu impactées par la présence des électrolytes. Ils fonctionnent sur une large gamme de pH et permettent de réaliser des gels transparents. Néanmoins la demanderesse a mis en évidence que les épaississants de l'invention présentaient l'inconvénient de contenir un ou plusieurs monomères contenant des groupes éthoxylés dont l'usage en cosmétique est de moins en moins bien perçu.

La demande de brevet européen publiée sous le numéro EP 0 562 344 et la demande de brevet français publiée sous le numéro 2 362 872 décrivent des copolymères à base notamment d'acide 2-acrylamido 2-méthyl 1-propanesulfonique salifié et de méthacrylate ou d'acrylate d'alkyle donc exempt de dérivés oxy-éthylénés. Mais ces copolymères, même s'ils sont des épaississants efficaces dans l'eau, perdent cette efficacité pas en présence de sels. Ils ne peuvent donc pas être utilisés en lieu et place des copolymères selon la demande de brevet français publiée sous le numéro 2 879 607.

La demande de brevet européen publiée sous le numéro EP 0 750 899 A2 divulgue un polymère amphiphile réticulé obtenu par polymérisation de 96,5% molaire d'acide 2-acrylamido 2-méthyl 1-propanesulfonique, 3,0% molaire de méthacrylate de lauryle, 0,5% molaire de méthylène bis(acrylamide) et son utilisation comme agent émulsionnant ou comme agent solubilisant.

La demande internationale publiée sous le numéro WO 2004/063228 A1 divulgue un polymère réticulé obtenu par polymérisation en émulsion inverse de 39% molaire d'acide 2-acrylamido 2-méthyl 1-propanesulfonique, 58,6% molaire d'acide acrylique, 0,4% molaire de méthacrylate de lauryle et 2% molaire de méthylène bis(acrylamide)et son utilisation comme agent épaississant.

La demande brevet européen publiée sous le numéro EP 1 138 703 A1 divulgue des polymères obtenus par polymérisation de 45% à 97% molaire d'acide 2-acrylamido 2-méthyl 1-propanesulfonique, partiellement ou totalement salifié, de 3% à 6% molaire de méthacrylate de stéaryle, de méthacrylate de lauryle ou d'acrylate d'hexadécyle, et optionnellement de 27% molaire à 52% d'acrylamide, seul ou en mélange avec de l'acrylate de 2-hydroxy éthyle, et leur utilisation comme épaississants.

Cependant les résultats en termes d'épaississement et/ou de tenue aux sels, ne sont pas satisfaisants, pour que ces polymères soient sélectionnables comme épaississants de formulations cosmétiques. La demande de brevet américain publiée sous le numéro US2007004851 A1 décrit des compositions émulsifiées réticulées, utilisées comme épaississant, obtenues par polymérisation d'acide 2-acrylamido 2-méthyl 1-propanesulfonique, de méthylène bis(acrylamide) et de méthacrylate de stéaryle ou de méthacrylate de lauryle.

La demande de brevet américain publiée sous le numéro US2011098364A1 et le brevet américain publié sous le numéro US6197287B1 décrivent des compositions émulsifiées réticulées utilisées comme épaississant ou émulsifiant, obtenues par polymérisation d'acide 2-acrylamido 2-méthyl 1-propanesulfonique, de méthylène bis(acrylamide) et d'acrylate de (2-hydroxy éthyle).

La demande de brevet européen publiée sous le numéro EP0503853A2 décrit des compositions émulsifiées réticulées, utilisées comme épaississant ou stabilisant, obtenues par polymérisation d'acide 2-acrylamido 2-méthyl 1-propanesulfonique, de méthylène bis(acrylamide) et d'acrylamide.

Les inventeurs se sont donc attachés à mettre au point un nouveau polyélectrolyte anionique qui après dispersion à 2% massique dans l'eau, forment un gel clair ayant une viscosité d'au moins 30.000 mPas mesurées à 20°C au moyen d'un viscosimètre Brookfield RVT à la vitesse de 5 tours par minute, stable sur une large gamme de pH et dont ladite viscosité mesurée dans les mêmes conditions se maintient après ajout de 2% massique de chlorure de sodium, à une valeur au moins égale à 50% de sa valeur mesurée dans les mêmes conditions en l'absence de sel.

C'est pourquoi l'invention a pour objet un polyélectrolyte anionique réticulé issu de la polymérisation pour 100% molaire d'unités monomériques issues d'un monomère possédant une fonction acide fort, partiellement salifiée ou totalement salifiée.

Par monomère comportant une fonction acide fort, on désigne notamment un monomère la fonction acide sulfonique ou la fonction acide phosphonique.

Dans le cadre de la présente invention, la fonction acide fort est partiellement ou totalement salifiée sous forme de sel de métal alcalin, tel que le sel de sodium ou sous forme de sel d'ammonium. L'invention a pour objet un polyélectrolyte anionique tel que défini précédemment, dans lequel les unités monomériques issues du monomère possédant une fonction acide fort, partiellement salifiée ou totalement salifiée, sont issues de l'acide 2-méthyl 2-[(1-oxo 2-propènyl) amino] 1-propanesulfonique partiellement salifié ou totalement salifié sous forme de sel de sodium ou de sel d'ammonium.

Par polymère réticulé, on désigne un polymère non linéaire se présentant à l'état de réseau tridimensionnel insoluble dans l'eau, mais gonflable à l'eau et conduisant donc à l'obtention d'un gel chimique.

Le polyélectrolyte anionique tel que défini précédemment est caractérisé en ce que l'agent de réticulation et/ou l'agent de ramification est le triacrylate de triméthylolpropane en proportion molaire supérieure ou égale à 0.5% molaire et inférieure ou égale à 5% molaire.

Le polyélectrolyte anionique réticulé tel que défini précédemment est issu de la polymérisation pour 100% molaire:
(i) - D'une proportion supérieure ou égale à 75% molaire et inférieure ou égale à 95% molaire d'unités monomériques issues de l'acide 2-méthyl 2-[(1-oxo2-propènyl)amino]1-propanesulfonique partiellement salifié ou totalement salifié sous forme de sel de sodium ou de sel d'ammonium;
(ii) - D'une proportion supérieure ou égale à 0,5% molaire et inférieure ou égale à 5% molaire d'unités monomériques issues du méthacrylate de lauryle de formule (I1), et du méthacrylate de stéaryle de formule (I2), dans un ratio molaire (I1)/(I2) supérieur ou égal à 1/6 et inférieur ou égal à 6/1; avec la formule (I1), correspondant à la formule (I) :

   CH2=CH(CH3)-C(=O)-OR1 (I)

   dans laquelle R1 représente un radical dodécyle et du méthacrylate de stéaryle de formule (I2), correspondant à la formule (I) dans laquelle R1 représente un radical octadécyle;
(iii) - D'une proportion supérieure ou égale à 0,5% molaire et inférieure ou égale à 3,0% molaire d'unités monomériques issues du triméthylol propanetriacrylate;
(iv) - Et d'une proportion molaire supérieure ou égale à 4,0% molaire et inférieure ou égale à 20% molaire d'unités monomériques issues d'acrylate de (2-hydroxy éthyle).

L'invention a plus particulièrement pour objet un polyélectrolyte anionique réticulé tel que défini précédemment, issu de la polymérisation pour 100% molaire :
(i) - D'une proportion supérieure ou égale à 83% molaire et inférieure ou égale à 90% molaire d'unités monomériques issues de l'acide 2-méthyl 2-[(1-oxo 2-propènyl) amino] 1-propanesulfonique partiellement salifié ou totalement salifié sous forme de sel de sodium ou de sel d'ammonium ;
(ii) - D'une proportion supérieure ou égal à 1,5% molaire et inférieure ou égale à 2,5% d'unités monomériques issues du méthacrylate de lauryle de formule (I₁), et du méthacrylate de stéaryle de formule (I₂), dans un ratio molaire. (I₁)/(I₂) supérieur ou égal à 1/6 et inférieur ou égal à 6/1 ;
(iii) - D'une proportion supérieure ou égale à 0,5% molaire et inférieure ou égale à 3,0% molaire d'unités monomériques issues du triméthylol propanetriacrylate ;
(iv) - De 8% molaire à 15% molaires d'unités monomériques issues d'acrylate de (2-hydroxy éthyle).

Le polyélectrolyte objet de la présente invention, peut également comprendre divers additifs, tels que des agents complexants, des agents de transfert ou des agents limiteurs de chaîne.

L'invention a aussi pour objet un procédé de préparation du polélectrolyte tel que défini précédemment, caractérisé en ce qu'il comprend :
- Une étape a) au cours de laquelle on mélange l'ensemble des monomères, un agent de neutralisation ammoniaqué du monomère possédant une fonction acide fort, l'agent réticulant et si nécessaire ou si désiré, les autres monomères et/ou additifs dans un solvant organique, de préférence le tert-butanol ;
- Une étape b) au cours de laquelle la réaction de polymérisation est amorcée par introduction d'un initiateur de radicaux libres dans la dispersion préparée à l'étape a), puis est laissée se dérouler jusqu'à sa complétude ;
- Optionnellement une étape c) d'échange de l'ion ammonium présent par l'ion sodium ou l'ion potassium ;
- Une étape d) de précipitation du polymère formé à l'issue de l'étape b), optionnellement de l'étape c) ;
- Optionnellement une étape e) de séchage du précipité obtenu à l'issue de

### l'étape d).

Comme agent de neutralisation ammoniaqué mis en oeuvre à l'étape a) du procédé tel que défini ci-dessus, il y a par exemple l'ammoniac ou encore l'hydrogénocarbonate d'ammonium.

A l'étape b) du procédé tel que défini ci-dessus, la réaction de polymérisation est amorcée à une température généralement égale ou supérieure à 50°C à l'aide d'un amorceur radicalaire produisant des radicaux par homolyse, tel que le peroxyde de dilauroyle, l'azo-bis(isobutyronitrile) ou les dérivés azoïques.

Selon une autre mise en oeuvre du procédé, tel que défini précédemment, la réaction de polymérisation est amorcée par un couple oxydo-réducteur.

A l'étape c) du procédé tel que défini ci-dessus, l'échange du cation ammonium par le cation sodium ou par le cation potassium est optionnellement effectuée avec du ter-butylate de sodium ou du ter-butylate de potassium.

A l'étape d) du procédé tel que défini ci-dessus, la précipitation du polyélectrolyte est effectuée soit par évaporation du solvant, soit par filtration du précipité.

L'invention a aussi pour objet l'utilisation du polyélectrolyte anionique tel que défini précédemment, comme épaississant et/ou comme stabilisant et/ou comme émulsionnant, d'une composition topique cosmétique, dermopharmaceutique ou pharmaceutique.

Le polyélectrolyte anionique selon l'invention est plus particulièrement utilisé comme agent suspensif de particules solides dans des compositions cosmétiques. Ces particules solides à suspendre peuvent revêtir différentes géométries, régulières ou irrégulières, et se présenter sous forme de perles, de billes, de tiges, de paillettes, de lamelles ou de polyèdres. Ces particules solides se caractérisent par un diamètre moyen apparent compris entre un micromètre et cinq millimètres, plus particulièrement entre dix micromètres et un millimètre.

Parmi les particules solides qui peuvent être mises en suspension par le polyélectrolyte selon l'invention dans des compositions cosmétiques, il y a les micas, l'oxyde de fer, l'oxyde de titane, l'oxyde de zinc, l'oxyde d'aluminium, le talc, la silice, le kaolin, les argiles, le nitrure de bore, le carbonate de calcium, le carbonate de magnésium, l'hydrogénocarbonate de magnésium, les pigments colorés inorganiques, les polyamides comme le nylon-6, les polyéthylènes, les polypropylènes, les polystyrènes, les polyesters, les polymères acryliques ou méthacryliques comme les polyméthylméthacrylates, le polytétrafluoroéthylène, les cires cristallines ou microcristallines, des sphères poreuses, le sulphide de sélénium, le pyrithione de zinc, les amidons, les alginates, les fibres de végétaux, les particules de Loofah, les particules d'éponges.

Pour optimiser ses propriétés suspensives de particules solides dans des compositions cosmétiques, on peut associer au polyélectrolyte anionique selon l'invention, des agents suspensifs couramment utilisés pour la préparation de compositions cosmétiques, comme des copolymères acryliques de faibles ou de moyens poids moléculaires.

L'invention a enfin pour objet une composition topique selon l'invention comporte habituellement entre 0,1% et 10% massique et plus particulièrement de 1% à 5% massique du polyélectrolyte anionique tel que défini précédemment. Le pH de la composition topique est de préférence supérieur ou égal à 3, plus particulièrement supérieur ou égal à 4,5, et encore plus particulièrement supérieur ou égal à 5,0.

L'expression "topique" signifie que la composition selon l'invention est mise en oeuvre par application sur la peau, les cheveux, le cuir chevelu ou les muqueuses, qu'il s'agisse d'une application directe ou d'une application indirecte lorsque la composition topique selon l'invention est imprégnée sur un support destiné à être mis en contact avec la peau (papier, lingette, textile, dispositif transdermique, etc.).

La composition topique selon l'invention peut se présenter sous toute forme physique, par exemple sous la forme d'un gel aqueux hydro-alcoolique ou hydro-glycolique ; d'une solution ; d'une poudre ; d'une suspension, d'une émulsion, d'une microémulsion ou d'une nano-émulsion, qu'elles soient de type eau-dans-huile, huile-dans-eau, eau-dans-huile-dans-eau ou huile-dans-eau-dans-huile.

La composition topique selon l'invention peut être conditionnée dans un flacon, dans un dispositif de type "flacon" pompe, sous forme pressurisée dans un dispositif aérosol, dans un dispositif muni d'une paroi ajourée comme une grille ou dans un dispositif muni d'un applicateur a billes (dit "roll-on").

De façon générale, la composition topique selon l'invention comporte également des additifs chimiques habituellement mis en oeuvre dans le domaine des formulations à usage topique, comme les tensioactifs moussants et/ou détergents, les tensioactifs épaississants et/ou gélifiants, les agents épaississants et/ou gélifiants, les agents stabilisants, les composés filmogènes, les solvants et co-solvants, les agents hydrotropes, les eaux thermales ou minérales les agents plastifiants, les agents émulsionnants et co-émulsionnants, les agents opacificants, les agents nacrants, les agents surgraissants, les séquestrants, les agents chélatants, les huiles, les cires, les agents antioxydants, les parfums, les huiles essentielles, les agents conservateurs, les agents conditionneurs, les agents déodorants, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes.

Comme exemples de tensioactifs moussants et/ou détergents, éventuellement présents dans la composition topique selon l'invention, on peut citer les tensioactifs moussants et/ou détergents anioniques, cationiques, amphotères ou non ioniques.

Parmi les tensioactifs anioniques moussants et/ou détergents que l'on peut mettre en oeuvre dans la composition topique selon l'invention, on peut citer les sels de métaux alcalins, de métaux alcalino-terreux, d'ammonium, d'amines, ou d'aminoalcools d'alkylethersulfates, d'alkylsulfates, d'alkylamidoéthersulfates, d'alkylarylpolyéthersulfates, de monoglycérides sulfates, d'alpha-oléfinesulfonates, de paraffinessulfonates, d'alkylphosphates, d'alkylétherphosphates, d'alkylsulfonates, d'alkylamidesulfonates, d'alkylarylsulfonates, d'alkylcarboxylates, d'alkylsulfosuccinates, d'alkyléthersulfosuccinates, d'alkylamidesulfosuccinates, d'alkylsulfoacétates, d'alkylsarcosinates, d'acyliséthionates, de N-acyltaurates, d'acyllactylates, de dérivés N-acylés d'acides aminés, de dérivés N-acylés de peptides, de dérivés N-acylés de protéines, de dérivés N-acylés d'acides gras.

Parmi les tensioactifs amphotères moussants et/ou détergents que l'on peut mettre en oeuvre dans la composition topique selon l'invention, on peut citer les alkylbétaïnes, les alkylamidobétaïnes, les sultaïnes, les alkylamidoalkylsulfobétaïnes, les dérivés d'imidazolines, les phosphobétaïnes, les amphopolyacétates et les amphopropionates.

Parmi les tensioactifs cationiques moussants et/ou détergents que l'on peut mettre en oeuvre dans la composition topique selon l'invention, on peut citer particulièrement les dérivés d'ammoniums quaternaires.

Parmi les tensioactifs non ioniques moussants et/ou détergents que l'on peut mettre en oeuvre dans la composition topique selon l'invention, on peut citer plus particulièrement les alkylpolyglycosides comportant un radical aliphatique, linéaire ou ramifié, saturé ou insaturé, et comportant de 8 à 16 atomes de carbone, comme l'octylpolyglucoside, le décylpolyglucoside, l'undécylénylpolyglucoside, le dodécylpolyglucoside, le tétradécylpolyglucoside, l'hexadécylpolyglucoside, le 1-12 dodécanediylpolyglucoside ; les dérivés d'huile de ricin hydrogénée éthoxylés comme le produit commercialisé sous le nom INCI « Peg-40 hydrogenated castor oil » ; les polysorbates comme le Polysorbate 20, le Polysorbate 40, le Polysorbate 60, le Polysorbate 70, le Polysorbate 80, le Polysorbate 85 ; les amides de coprah ; les N-alkylamines.

Dans les compositions topiques selon l'invention comprenant en outre au moins un agent tensioactif moussant, le rapport massique entre ledit agent tensioactif moussant et le polyélectrolyte anionique selon l'invention est supérieur ou égal à 1/10 et inférieur ou égal à 40/1, plus particulièrement supérieur ou égal à 1/1 et inférieur ou égal à 40/1, et encore plus particulièrement supérieur ou égal à 4/1 et inférieur ou égal à 40/1. La combinaison d'au moins un agent tensioactif moussant, tel que défini ci-dessus et du polyélectrolyte anionique selon l'invention permet d'obtenir des compositions topiques qui produisent un faible volume de mousse et dont les bulles d'air la composant sont stables et de taille suffisamment fines pour limiter les risque d'irritation oculaire lors de la l'application sur le visage. De telles compositions topiques selon l'invention comportent pour 100% de leur masse :
- De 0,1% à 5% massique du polyélectrolyte anionique selon l'invention,
- De 10% à 50% massique d'au moins un agent tensioactif moussant,
- De 0,01% à 10 % massique d'au moins un agent acide (A) sélectionné parmi le groupe constitué par les α-hydroxy acides et les β-hydroxy acides, libres, partiellement ou totalement salifiés, et
- De 89,89% à 35% d'eau.

Comme exemples de tensioactifs épaississants et/ou gélifiants, éventuellement présents dans la composition topique selon l'invention, on peut citer les esters gras d'alkylpolyglycosides éventuellement alcoxylés, comme les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120 ; les esters gras alcoxylés tels que le PEG 150 pentaérythrytyl tétrastéarate commercialisé sous l'appellation CROTHIX™ DS53, le PEG 55 propylène glycol oléate commercialisé sous l'appellation ANTIL™ 141 ; les carbamates de polyalkylène glycols à chaînes grasses comme le PPG-14 laureth isophoryl dicarbamate commercialisé sous l'appellation ELFACOS™ T211, le PPG-14 palmeth-60 hexyl dicarbamate commercialisé sous l'appellation ELFACOS™ GT2125.

Comme exemples d'agents épaississants et/ou gélifiants éventuellement présents dans la composition topique selon l'invention, on peut citer les polymères de type polyélectrolytes, linéaires ou branchés ou réticulés, comme l'homopolymère de l'acide acrylique, l'homopolymère de l'acide méthacrylique, l'homopolymère de l'acide-2-méthyl-[(1-oxo-2-propényl)amino]-1-propanesulfonique (AMPS), les copolymères de l'acide acrylique et de l'AMPS, les copolymères de l'acrylamide et de l'AMPS, les copolymères de la vinylpyrolidone et de l'AMPS, les copolymères de l'AMPS et de l'acrylate de (2-hydroxyéthyle), les copolymères de l'AMPS et du méthacrylate de (2-hydroxyéthyle), les copolymères de l'AMPS et de l'hydroxyéthylacrylamide, les copolymères de l'AMPS et du N,N-diméthyl acrylamide, les copolymères de l'AMPS et du tris(hydroxy- methyl)acrylamido methane (THAM), les copolymères de l'acide acrylique ou méthacrylique et de l'acrylate de (2-hydroxy éthyle), les copolymères de l'acide acrylique ou méthacrylique et du méthacrylate de (2-hydroxy éthyle), les copolymères de l'acide acrylique ou méthacrylique et de l'hydroxyéthylacrylamide, les copolymères de l'acide acrylique ou méthacrylique et du THAM, les copolymères de l'acide acrylique ou méthacrylique et du N,N-diméthyl acrylamide, les terpolymères de l'acide acrylique ou méthacrylique, de l'AMPS et de l'acrylate de (2-hydroxy éthyle), les terpolymères de l'acide acrylique ou méthacrylique, de l'AMPS et du méthacrylate de (2-hydroxy éthyle), les terpolymères de l'acide acrylique ou méthacrylique, de l'AMPS et du THAM, les terpolymères de l'acide acrylique ou méthacrylique, de l'AMPS et du N,N-diméthyl acrylamide, les terpolymères de l'acide acrylique ou méthacrylique, de l'AMPS et de l'acrylamide, les copolymères de l'acide acrylique ou de l'acide méthacrylique et d'acrylates d'alkyle dont la chaîne carbonée comprend entre quatre et trente atomes de carbone et plus particulièrement entre dix et trente atomes de carbone, les copolymères de l'AMPS et d'acrylates d'alkyle dont la chaîne carbonée comprend entre quatre et trente atomes de carbone et plus particulièrement entre dix et trente atomes de carbone. Les polymères de type polyélectrolytes, linéaires ou branchés ou réticulés éventuellement présents dans la composition topique selon l'invention peuvent se présenter sous la forme d'une solution, d'une suspension aqueuse, d'une émulsion eau-dans-huile, d'une émulsion huile-dans-eau, d'une poudre. Les polymères de type polyélectrolytes, linéaires ou branchés ou réticulés éventuellement présents dans la composition topique selon l'invention peuvent être sélectionnés parmi les produits commercialisés sous les appellations SIMULGEL™ EG, SIMULGEL™EPG, SEPIGEL™ 305, SIMULGEL™ 600, SIMULGEL™ NS, SIMULGEL™ INS 100, SIMULGEL™ FL, SIMULGEL™ A, SIMULGEL™ SMS 88, SEPINOV™EMT 10, SEPIPLUS™400, SEPIPLUS™265, SEPIPLUS™S, ARISTOFLEX™AVC, ARISTOFLEX™AVS, NOVEMER™EC-1, FLOCARE™ET 25, FLOCARE™ET 75, FLOCARE™ET 26, FLOCARE™ET 30, FLOCARE™ET 58, FLOCARE™PSD 30, VISCOLAM™AT 64, VISCOLAM™AT 100.

Comme exemples d'agents épaississants et/ou gélifiants éventuellement présents dans la composition topique selon l'invention, on peut citer les polysaccharides constitués uniquement d'oses, comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, comme les galactomannanes provenant de la gomme de cassia (DS = 1/5), de la gomme de caroube (DS = 1/4), de la gomme de tara (DS = 1/3), de la gomme de guar (DS = 1/2), de la gomme de fenugrec (DS = 1).

Comme exemples d'agents épaississants et/ou gélifiants éventuellement présents dans la composition topique selon l'invention, on peut citer les polysaccharides constitués de dérivés d'oses, comme les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme de arabique et de gomme de karaya, les glucosaminoglycanes.

Dans les compositions topiques selon l'invention comprenant en outre au moins un polysaccharide choisi parmi le groupe constitué par un galactomannane, la gomme arabique et la gomme de xanthane, le rapport massique entre ledit polysaccharide et le polyélectrolyte anionique selon l'invention est supérieur ou égal à 1/3 et inférieur ou égal à 3/1, plus particulièrement ou égal à 1/2 et inférieur ou égal à 3/2. La combinaison du polysaccharide, tel que défini ci-dessus, et du polyélectrolyte anionique selon l'invention permet d'obtenir des compositions topiques selon l'invention se présentant sous forme d'émulsions, riches en sels, conservant une viscosité élevée et un aspect homogène après une période de stockage prolongée. De telles compositions topiques selon l'invention peuvent comprendre pour 100% de leur masse de 0,1% à 25% massique d'au moins un sel et peuvent présenter une viscosité dynamique mesurée à 20°C, au moyen d'un viscosimètre Brookfield, supérieure ou égale à 30 000 mPa.s et inférieure ou égale à 200 000 mPa.s.

Comme exemples d'agents épaississants et/ou gélifiants éventuellement présents dans la composition topique selon l'invention, on peut citer la cellulose, les dérivés de cellulose comme la méthyl-cellulose, l'éthyl-cellulose, l'hydroxypropyl cellulose, les silicates, l'amidon, les dérivés hydrophiles de l'amidon, les polyuréthanes.

Comme exemples d'agents stabilisants éventuellement présents dans la composition topique selon l'invention, on peut citer par exemple les cires microcristallines, et plus particulièrement l'ozokérite, les sels minéraux tels que le chlorure de sodium ou le chlorure de magnésium, les polymères siliconés tels que les copolymères polysiloxane polyalkyl polyéther.

Comme exemples de solvants éventuellement présents dans la composition topique selon l'invention, on peut citer l'eau, les solvants organiques comme le glycérol, le diglycérol, les oligomères du glycérol, l'éthylène glycol, le propylène glycol, le butylène glycol, le 1,3-propanediol, le 1,2-propanediol, l'hexylèneglycol, le diéthylèneglycol, le xylitol, l'érythritol, le sorbitol, les alcools hydrosolubles tels que l'éthanol, l'isopropanol ou le butanol, les mélanges d'eau et desdits solvants organiques.

Comme exemples d'eaux thermales ou minérales éventuellement présentes dans la composition topique selon l'invention, on peut citer les eaux thermales ou minérales ayant une minéralisation d'au moins 300 mg/l, en particulier l'eau d'Avene, l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizieres, l'eau de Neyrac-les-bains, l'eau de Lons le Saunier, l'eau de Rochefort, l'eau de Saint Christau, l'eau des Fumades et l'eau de Tercis-les-bains.

Comme exemples d'agents hydrotropes éventuellement présents dans la composition topique selon l'invention, on peut citer les xylènes sulfonates, les cumènes sulfonates, l'hexylpolyglucoside, le 2-éthylhexylpolyglucoside, le n-heptylpolyglucoside.

Comme exemples d'agents tensioactifs émulsionnants éventuellement présents dans la composition topique selon l'invention, on peut citer les tensioactifs non ioniques, des tensioactifs anioniques, des tensioactifs cationiques.

Comme exemples de tensioactifs non-ioniques émulsionnants éventuellement présents dans la composition topique selon l'invention, on peut citer les esters d'acides gras et de sorbitol, comme les produits commercialisés sous les appellations MONTANE™40, MONTANE™60, MONTANE™70, MONTANE™80 et MONTANE™85 ; les compositions comprenant du stéarate de glycérol et l'acide stéarique éthoxylé entre 5 moles et 150 moles d'oxyde d'éthylène, comme la composition comprenant de l'acide stéarique éthoxylé à 135 moles d'oxyde d'éthylène et du stéarate de glycérol commercialisée sous l'appellation SIMULSOL™ 165 ; les esters de mannitan ; les esters de mannitan éthoxylés ; les esters de sucrose ; les esters de méthylglucoside ; les alkylpolyglycosides comportant un radical aliphatique, linéaire ou ramifié, saturé ou insaturé, et comportant de 14 à 36 atomes de carbone, comme le tétradécylpolyglucoside, l'hexadécylpolyglucoside, l'octadécylpolyglucoside, l'hexadécylpolyxyloside, l'octadécylpolyxyloside, l'eicosylpolyglucoside, le dodécosylpolyglucoside, le 2-octyldodécylpolyxyloside, le 12-hydroxystéarylpolyglucoside ; les compositions d'alcools gras linéaires ou ramifiés, saturés ou insaturés, et comportant de 14 à 36 atomes de carbone, et d' alkylpolyglycosides tels que décrits précédemment.

Comme exemples de tensioactifs non-ioniques éventuellement présents dans la composition topique selon l'invention, on peut citer le glycéryl stéarate citrate, le cétéarylsulfate, les savons comme le stéarate de sodium ou le stéarate de triéthanolammonium, les dérivés N-acylés d'acides aminés salifiés comme par exemple le stéaroyl glutamate.

Comme exemples de tensioactifs cationiques émulsionnants éventuellement présents dans la composition topique selon l'invention, on peut citer les aminoxydes, le quaternium-82 et les tensioactifs décrits dans la demande de brevet WO96/00719 et principalement ceux dont la chaîne grasse comprend au moins 16 atomes de carbone.

Comme exemples d'agents opacifiants et/ou nacrants éventuellement présents dans la composition topique selon l'invention, on peut citer le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras comportant de 12 à 22 atomes de carbone.

Comme exemples d'agents de texture éventuellement présents dans la composition topique selon l'invention, on peut citer des dérivés N-acylés d'acides aminés, comme la lauroyl lysine commercialisée sous l'appellation AMINOHOPE™LL, l'octenyl starch succinate commercialisé sous l'appellation DRYFLO™, le myristyl polyglucoside commercialisé sous l'appellation MONTANOV™ 14, les fibres de cellulose, les fibres de coton, les fibres de chitosane, le talc, la séricite, le mica.

Comme exemples d'agents déodorants éventuellement présents dans la composition topique selon l'invention, on peut citer par exemple les silicates alcalins, les sels de zinc comme le sulfate de zinc, le gluconate de zinc, le chlorure de zinc, le lactate de zinc ; les sels d'ammonium quaternaires comme les sels de cétyltriméthylammonium, les sels de cétylpyridinium ; les dérivés du glycérol comme le caprate de glycérol , le caprylate de glycérol, le caprate de polyglycérol ; le 1,2 décanediol ; le 1,3 propanediol ; l'acide salicylique ; le bicarbonate de sodium ; les cyclodextrines ; les zéolithes métalliques ; le TRICLOSAN™ ; le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples d'huiles éventuellement présentes dans la composition topique selon l'invention, on peut citer les huiles minérales telles que l'huile de paraffine, l'huile de vaseline, les isoparaffines ou les huiles blanches minérales ; les huiles d'origine animale, telles que le squalène ou le squalane ;les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes les huiles végétales éthoxylées ; les huiles synthétiques comme les esters d'acides gras tels que le myristate de butyle, le myristate de propyle, le myristate d'isopropyle, le myristate de cétyle, le palmitate d'isopropyle, le palmitate d'octyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les poly(alpha-oléfine), les polyoléfines comme le poly(isobutane), les isoalcanes de synthèse comme l'isohexadécane, l'isododécane, les huiles perfluorées ; les huiles de silicone comme les diméthylpolysiloxanes, les méthylphényl - polysiloxanes, les silicones modifiées par des aminés, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles. Par « huiles », on entend dans la présente demande les composés et/ou les mélanges de composés insolubles dans l'eau, se présentant sous un aspect liquide à une température de 25°C.

Comme exemples de cires éventuellement présentes dans la composition topique selon l'invention, on peut citer la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène ; les cires de silicone ; les cires végétales ; les alcools gras et les acides gras solides à température ambiante ; les glycérides solides à température ambiante. Par « cires », on entend dans la présente demande les composés et/ou les mélanges de composés insolubles dans l'eau, se présentant sous un aspect solide à une température supérieure ou égale à 45°C.

Comme exemples de principes actifs éventuellement présents dans la composition topique selon l'invention, on peut citer les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés) ; les composés montrant une action éclaircissante ou dépigmentante de la peau comme le ω-undecelynoyl phénylalanine commercialisé sous l'appellation SEPIWHITE™ MSH, le SEPICALM™VG, le mono ester et/ou le diester de glycérol du ω-undecelynoyl phénylalanine, les ω-undecelynoyl dipeptides, l'arbutine, l'acide kojique, l'hydroquinone ; les composés montrant une action apaisante notamment le SEPICALM™ S, l'allantoïne et le bisabolol ; les agents anti-inflammatoires ; les composés montrant une action hydratante comme l'urée, les hydroxyurées, le glycérol, les polyglycérols, le glycérolglucoside, le diglycérolglucoside, les polyglycérylglucosides, le xylitylplucoside ; les extraits végétaux riches en polyphénols comme les extraits de raisin, les extraits de pin, les extraits de vin, les extraits d'olives ; les composés montrant une action amincissante ou lipolytique comme la caféine ou ses dérivés, l'ADIPOSLIM™, l'ADIPOLESS™, la fucoxanthine ; les protéines N-acylées ; les peptides N-acylés comme le MATRIXIL™ ; les acides aminés N-acylés ; les hydrolysâts partiels de protéines N-acylés ; les acides aminés ; les peptides ; les hydrolysâts totaux de protéines ; les extraits de soja, par exemple la Raffermine™ ; les extraits de blé par exemple la TENSINE™ ou la GLIADINE™ ; les extraits végétaux, tels que les extraits végétaux riches en tanins, les extraits végétaux riches en isoflavones ou les extraits végétaux riches en terpènes ; les extraits d'algues d'eau douce ou marines ; les extraits de plantes marines ; les extraits marins en général comme les coraux ; les cires essentielles ; les extraits bactériens ; les céramides ; les phospholipides ; les composés montrant une action antimicrobienne ou une action purifiante, comme le LIPACIDE™ C8G, le LIPACIDE™ UG, le SEPICONTROL™ A5 ; l'OCTOPIROX™ ou le SENSIVA™ SC50 ; les composés montrant une propriété énergisante ou stimulante comme le PHYSIOGENYL™, le panthénol et ses dérivés comme le SEPICAP™ MP ; les actifs anti-âge comme le SEPILIFT™ DPHP, le LIPACIDE™ PVB, le SEPIVINOL™, le SEPIVITAL™, le MANOLIVA™, le PHYTO-AGE™, le TIMECODE™ ; le SURVICODE™ ; les actifs anti-photo vieillissement ; les actifs protecteurs de l'intégrité de la jonction dermo-épidermique ; les actifs augmentant la synthèse des composants de la matrice extracellulaire comme le collagène, les élastines, les glycosaminoglycanes ; les actifs agissant favorablement sur la communication cellulaire chimique comme les cytokines ou physiques comme les intégrines ; les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés) ; les actifs améliorant la microcirculation cutanée, par exemple les veinotoniques ; les actifs drainants ; les actifs à visée décongestionnante comme les extraits de ginko biloba, de lierre, de marron d'inde, de bambou, de ruscus, de petit houx, de *centalla asiatica,* de fucus, de romarin, de saule ; les agents de bronzage ou de brunissement de la peau, comme la dihydroxyacétone, l'isatine, l'alloxane, la ninhydrine, le glycéraldéhyde, l'aldéhyde mésotartrique, la glutaraldéhyde, l'érythrulose.

Comme exemples d'agents antioxydants éventuellement présents dans la composition topique selon l'invention, on peut citer l'EDTA et ses sels, l'acide citrique, l'acide tartarique, l'acide oxalique, le BHA (butylhydroxyanisol), le BHT (butylhydroxytoluène), les dérivés de tocophérol tels que l'acétate de tocophérol, des mélanges de composés antioxydants tels que la DISSOLVINE™ GL 47S commercialisé par la société Akzo Nobel sous le le nom INCI : Tetrasodium Glutamate Diacetate.

Comme exemples de filtres solaires éventuellement présents dans la composition topique selon l'invention, on peut citer tous ceux figurant dans la directive cosmétique 76/768/CEE modifiée annexe VII. Parmi les filtres organiques solaires éventuellement présents dans la composition topique selon l'invention, on peut citer la famille des dérivés de l'acide benzoïque comme les acides para-aminobenzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA; la famille des dérivés de l'acide anthranilique comme l'homomenthyl-N-acétyl anthranilate ; la famille des dérivés de l'acide salicylique comme le salicylate d'amyle, le salicylate d'homomenthyle, le salicylate d'éthylhexyle, le salicylate de phényle, le salicylate de benzyle, le salicylate de p-isopropanolphényle ; la famille des dérivés de l'acide cinnamique comme le cinnamate d'éthylhexyle, le cinnamate d'éthyl-4-isopropyle, le cinnamate de méthyl-2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de p-méthoxy 2-éthylhexyle), le cinnamate de p-méthoxy 2-éthoxyéthyle, le cinnamate de p-méthoxycyclohexyle, le cinnamate d'éthyl-α-cyano-β-phényle, le cinnamate de 2-éthylhexyl-□α-cyano-β-phényle, le cinnamate de diparaméthoxy mono-2-éthylhexanoyl de glycéryle ; la famille des dérivés de la benzophénone comme la 2,4-dihydroxybenzophénone, la 2,2'-dihydroxy-4-méthoxybenzophénone, la 2,2',4,4'-tétrahydroxybenzophénone, la 2-hydroxy-4-méthoxybenzophénone, la 2-hydroxy-4-méthoxy-4'-méthylbenzophénone, la 2-hydroxy-4-méthoxybenzophénone-5-sulfonate, la 4-phénylbenzophénone, le 2-éthylhexyl-4'-phénylbenzophénone-2-carboxylate, la 2-hydroxy-4-n-octyloxybenzophénone, la 4-hydroxy-3-carboxybenzophénone ; le 3-(4'-méthylbenzylidène)-d,l-camphre, le 3-(benzylidène)-d,l-camphre, le benzalkonium méthosulfate camphre ; l'acide urocanique, l'urocanate d'éthyle ; la famille des dérivés de l'acide sulfonique comme l'acide sulfonique 2-phénylbenzimidazole-5 et ses sels ; la famille des dérivés de la triazine comme l'hydroxyphényl triazine, l'éthylhexyloxyhydroxyphényl-4-méthoxyphényltriazine, le 2,4,6-trianillino-(p-carbo-2'-éthylhexyl-1'-oxy)-1,3,5-triazine, le 4,4-((6-(((1,1-diméthyléthyl) amino)carbonyl)phenyl)amino)-1,3,5-triazine-2,4-diyl diimino) bis-(2-éthylhexyl) ester de l'acide benzoïque, le 2-phényl-5-méthylbenzoxazole, le 2,2'-hydroxy-5-méthylphénylbenzotriazole, le 2-(2'-hydroxy-5'-t-octylphényl)benzotriazole, le 2-(2'-hydroxy-5'-méthyphényl)benzotriazole; la dibenzazine; le dianisoylméthane, le 4-méthoxy-4"-t-butylbenzoylméthane ; la 5-(3,3-diméthyl-2-norbornylidène)-3-pentan-2-one ; la famille des dérivés du diphénylacrylate comme le 2-éthylhexyl-2-cyano-3,3-diphényl-2-propènoate, l'éthyl-2-cyano-3,3-diphényl-2-propènoate ; la famille des polysiloxanes comme le malonate de benZylidène siloxane.

Parmi les filtres inorganiques solaires, également appelés "écrans minéraux", éventuellement présents dans la composition topique selon l'invention, on peut citer les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

La composition topique selon l'invention peut être plus particulièrement une composition parfumante, présentant un aspect translucide ou transparent ou blanchâtre, stables au stockage dans le temps et n'altérant pas les propriétés olfactives des parfums mis en oeuvre, comprenant pour 100% de leur masse :
- De 0,1% à 40% massique, plus particulièrement de 0.1% à 20% massique, plus particulièrement de 1% à 12% massique, et encore plus particulièrement de 1% à 10% massique d'au moins une substance parfumante ou aromatisante,
- De 5 % à 90% massique, plus particulièrement de 20 % à 70% massique d'au moins un solvant volatil,
- De 0,1 % à 5% massique, plus particulièrement de 0,1% à 3.5% massique, et plus particulièrement de 0,5% à 3% massique, et encore plus particulièrement entre 0,8% et 2% massique d'au moins un polyélectrolyte anionique selon l'invention,
- De 94,8% à 5% massique d'eau, optionnellement,
- De 2% à 20% massique d'au moins une huile et/ou une cire, telle que définie précédemment.

Par « substance parfumante ou aromatisante», on désigne une substance d'origine naturelle ou synthétique susceptible de dégager une odeur, plus ou moins persistante.

La composition topique selon l'invention peut être plus particulièrement une suspension de particules solides, comprenant pour 100% de sa masse :
- De 0,1% à 5% massique, plus particulièrement de 0,15% à 2,5% massique d'au moins un polyélectrolyte anionique selon l'invention,
- De 0,05% à 2% massique, plus particulièrement de 0,15% à 2,5% massique d'au moins une particule solide.

Les particules solides suspendues présentes dans la composition topique selon l'invention, sont précédemment décrites dans la présente demande. Selon la nature des particules solides, la composition topique selon l'invention comprenant lesdites particules solides en suspension peut être utilisée comme gel exfoliant de la peau du corps et du visage, comme gel de soin anti-âge de la peau lorsque les particules suspendues sont des microsphères comprenant des actifs cosmétiques hydratant, comme des microsphères comprenant de l'acide hyaluronique.

La composition topique selon l'invention peut être plus particulièrement une composition de maquillage destinée à être appliquée sur la peau, sur les sourcils, sur les ongles, comme une composition de fond de teint, une composition de mascara, une composition de fard à paupière. L'utilisation du polyélectrolyte selon l'invention pour préparer une composition de mascara selon l'invention, permet d'enlever plus aisément le mascara des cils lors du démaquillage.

Les exemples suivants illustrent l'invention sans toutefois la limiter.

### Exemple 1 (selon l'invention) : Polyélectrolyte réticulé de 2-méthyl 2-[(1-oxo 2-propènyl) amino] 1-propanesulfonate d'ammonium, d'acrylate de (2-hydroxy éthyle), de méthacylate de stéaryle et de méthacrylate de lauryle, réticulé au triacrylate de triméthylolpropane (ATBS/HEA/MAS/MALAU : 88,1/9,9/1,5/0,5 ; Polyélectrolyte 1).

On charge dans un réacteur maintenu à 25°C sous agitation contenant 245 g de tertio butanol :
- 33,8g d'acide 2-acrylamido 2- méthyl propanesulfonique (ATBS) ;
- 12,9g d'hydrogénocarbonate d'ammonium ;
- 2,12g d'acrylate de (2-hydroxy éthyle) (HEA) ;
- 0,93g de méthacrylate de stéaryle (MAS) ;
- 0,24g de méthacrylate de lauryle (MALAU) ;
- 0,54g de triacrylate de triméthylol propane (TMPTA).

Après un temps suffisant pour atteindre une bonne homogénéisation de la solution, on désoxygène par barbotage d'azote puis on porte la température du milieu à 70°C. Lorsque la température désirée est atteinte, 0,50 g de peroxyde de dilauroyle sont ajoutés. La polymérisation démarre instantanément. On maintient alors le milieu réactionnel pendant environ 60 minutes à cette température, puis on chauffe à 80°C. Cette température est maintenue 2 heures avant de refroidir. La poudre qui s'est formée en cours de polymérisation est filtrée et séchée pour obtenir le polyélectrolyte 1.

### Exemple 2 (selon l'invention) : Polyélectrolyte réticulé de 2-méthyl 2-[(1-oxo 2-propènyl) amino] 1-propanesulfonate d'ammonium, d'acrylate de (2-hydroxy éthyle), de méthacylate de stéaryle et de méthacrylate de lauryle, réticulé au triacrylate de triméthylolpropane (ATBS/HEA/MAS/MALAU : 88,1/9,9/1,0/1,0 ; Polyélectrolyte 2).

On charge dans un réacteur maintenu à 25°C sous agitation contenant 245 g de tertio butanol :
- 33,8g d'acide 2-acrylamido 2-méthyl propanesulfonique (ATBS) ;
- 12,9g d'hydrogénocarbonate d'ammonium ;
- 2,12g d'acrylate de (2-hydroxy éthyle) (HEA) ;
- 0,62g de méthacrylate de stéaryle (MAS) ;
- 0,48g de méthacrylate de lauryle (MALAU) ;
- 0,54g de triacrylate de triméthylol propane (TMPTA).

Après un temps suffisant pour atteindre une bonne homogénéisation de la solution, on désoxygène par barbotage d'azote puis on porte la température du milieu à 70°C. Lorsque la température désirée est atteinte, 0,50 g de peroxyde de dilauroyle sont ajoutés. La polymérisation démarre instantanément. On maintient alors le milieu réactionnel pendant environ 60 minutes à cette température, puis on chauffe à 80°C. Cette température est maintenue 2 heures avant de refroidir. La poudre qui s'est formée en cours de polymérisation est filtrée et séchée pour obtenir le polyélectrolyte 2.

### Exemple 3 (selon l'invention) : Polyélectrolyte réticulé de 2-méthyl 2-[(1-oxo 2-propènyl) amino] 1-propanesulfonate d'ammonium, d'acrylate de (2-hydroxy éthyle), de méthacylate de stéaryle et de méthacrylate de lauryle, réticulé au triacrylate de triméthylolpropane (ATBS/HEA/MAS/MALAU : 88,1/9,9/0,7/1,3 ; Polyélectrolyte 3).

On charge dans un réacteur maintenu à 25°C sous agitation contenant 245 g de tertio butanol :
- 33,8g d'acide 2-acrylamido 2-méthyl propanesulfonique (ATBS) ;
- 12,9g d'hydrogénocarbonate d'ammonium ;
- 2,12g d'acrylate de (2-hydroxy éthyle) (HEA) ;
- 0,46g de méthacrylate de stéaryle (MAS) ;
- 0,60g de méthacrylate de lauryle (MALAU) ;
- 0,54g de triacrylate de triméthylol propane (TMPTA).

Après un temps suffisant pour atteindre une bonne homogénéisation de la solution, on désoxygène par barbotage d'azote puis on porte la température du milieu à 70°C. Lorsque la température désirée est atteinte, 0,50 g de peroxyde de dilauroyle sont ajoutés. La polymérisation démarre instantanément. On maintient alors le milieu réactionnel pendant environ 60 minutes à cette température, puis on chauffe à 80°C. Cette température est maintenue 2 heures avant de refroidir. La poudre qui s'est formée en cours de polymérisation est filtrée et séchée pour obtenir le polyélectrolyte 3.

### Exemple 4 (selon l'invention) : Polyélectrolyte réticulé de 2-méthyl 2-[(1-oxo 2-propènyl)amino]1-propanesulfonate d'ammonium, d'acrylate de (2-hydroxy éthyle), de méthacylate de stéaryle et de méthacrylate de lauryle, réticulé au triacrylate de triméthylolpropane (ATBS/HEA/MAS/MALAU : 88,1/9,9/0,3/1,7 ; Polyélectrolyte 4).

On charge dans un réacteur maintenu à 25°C sous agitation contenant 245 g de tertio butanol :
- 33,8g d'acide 2-acrylamido 2- méthyl propanesulfonique (ATBS) ;
- 12,9g d'hydrogénocarbonate d'ammonium ;
- 2,12g d'acrylate de (2-hydroxy éthyle) (HEA) ;
- 0,19g de méthacrylate de stéaryle (MAS) ;
- 0,82g de méthacrylate de lauryle (MALAU) ;
- 0,54g de triacrylate de triméthylol propane (TMPTA).

Après un temps suffisant pour atteindre une bonne homogénéisation de la solution, on désoxygène par barbotage d'azote puis on porte la température du milieu à 70°C. Lorsque la température désirée est atteinte, 0,50 g de peroxyde de dilauroyle sont ajoutés. La polymérisation démarre instantanément. On maintient alors le milieu réactionnel pendant environ 60 minutes à cette température, puis on chauffe à 80°C. Cette température est maintenue 2 heures avant de refroidir. La poudre qui s'est formée en cours de polymérisation est filtrée et séchée pour obtenir le polyélectrolyte 4.

### Exemple 5 (selon l'invention) : Polyélectrolyte réticulé de 2-méthyl 2-[(1-oxo 2-propènyl) amino] 1-propanesulfonate d'ammonium, d'acrylate de (2-hydroxy éthyle), de méthacylate de stéaryle et de méthacrylate de lauryle, réticulé au triacrylate de triméthylolpropane (ATBS/HEA/MAS/MALAU : 86,4/9,7/1,9/2,0, ; Polyélectrolyte 5).

On charge dans un réacteur maintenu à 25°C sous agitation contenant 245 g de tertio butanol :
- 33,8g d'acide 2-acrylamido 2- méthyl propanesulfonique (ATBS) ;
- 12,9g d'hydrogénocarbonate d'ammonium ;
- 2,12g d'acrylate de (2-hydroxy éthyle) (HEA) ;
- 1,24g de méthacrylate de stéaryle (MAS) ;
- 0,96g de méthacrylate de lauryle (MALAU) ;
- 0,54g de triacrylate de triméthylol propane (TMPTA).

Après un temps suffisant pour atteindre une bonne homogénéisation de la solution, on désoxygène par barbotage d'azote puis on porte la température du milieu à 70°C. Lorsque la température désirée est atteinte, 0,50 g de peroxyde de dilauroyle sont ajoutés. La polymérisation démarre instantanément. On maintient alors le milieu réactionnel pendant environ 60 minutes à cette température, puis on chauffe à 80°C. Cette température est maintenue 2 heures avant de refroidir. La poudre qui s'est formée en cours de polymérisation est filtrée et séchée pour obtenir le polyélectrolyte 5.

### Exemple 6 (référence) : Polyélectrolyte réticulé de 2-méthyl 2-[(1-oxo 2-propènyl) amino] 1-propanesulfonate d'ammonium, d'acrylate de (2-hydroxy éthyle), et de méthacylate de stéaryle, réticulé au triacrylate de triméthylolpropane (ATBS/HEA/MAS : 88,2/9,8/2 ; Polyélectrolyte 6).

On charge dans un réacteur maintenu à 25°C sous agitation contenant 245 g de tertio butanol :
- 33,8g d'acide 2-acrylamido 2- méthyl propanesulfonique (ATBS) ;
- 12,9g d'hydrogénocarbonate d'ammonium ;
- 2,12g d'acrylate de (2-hydroxy éthyle) (HEA) ;
- 1,24g de méthacrylate de stéaryle (MAS) ;
- 0,54g de triacrylate de triméthylol propane (TMPTA).

Après un temps suffisant pour atteindre une bonne homogénéisation de la solution, on désoxygène par barbotage d'azote puis on porte la température du milieu à 70°C. Lorsque la température désirée est atteinte, 0,50 g de peroxyde de dilauroyle sont ajoutés. La polymérisation démarre instantanément. On maintient alors le milieu réactionnel pendant environ 60 minutes à cette température, puis on chauffe à 80°C. Cette température est maintenue 2 heures avant de refroidir. La poudre qui s'est formée en cours de polymérisation est filtrée et séchée pour obtenir le polyélectrolyte 6.

### Exemple 7 (référence) : Polyélectrolyte réticulé de 2-méthyl 2-[(1-oxo 2-propènyl) amino] 1-propanesulfonate d'ammonium, d'acrylate de (2-hydroxy éthyle), et de méthacrylate de lauryle, réticulé au triacrylate de triméthylolpropane (ATBS/HEA/MALAU : 88,1/9,9/2,0 ; Polyélectrolyte 7).

On charge dans un réacteur maintenu à 25°C sous agitation contenant 245 g de tertio butanol :
- 33,8g d'acide 2-acrylamido 2- méthyl propanesulfonique (ATBS) ;
- 12,9g d'hydrogénocarbonate d'ammonium ;
- 2,12g d'acrylate de (2-hydroxy éthyle) (HEA) ;
- 0,96g de méthacrylate de lauryle (MALAU) ;
- 0,54g de triacrylate de triméthylol propane (TMPTA).

Après un temps suffisant pour atteindre une bonne homogénéisation de la solution, on désoxygène par barbotage d'azote puis on porte la température du milieu à 70°C. Lorsque la température désirée est atteinte, 0,50 g de peroxyde de dilauroyle sont ajoutés. La polymérisation démarre instantanément. On maintient alors le milieu réactionnel pendant environ 60 minutes à cette température, puis on chauffe à 80°C. Cette température est maintenue 2 heures avant de refroidir. La poudre qui s'est formée en cours de polymérisation est filtrée et séchée pour obtenir le polyélectrolyte 7.

### Exemple 8 (selon l'invention) : Polyélectrolyte réticulé de 2-méthyl 2-[(1-oxo 2-propènyl) amino] 1-propanesulfonate de sodium, d'acrylate de (2-hydroxy éthyle), de méthacylate de stéaryle et de méthacrylate de lauryle, réticulé au triacrylate de triméthylolpropane (ATBS/HEA/MAS/MALAU : 88,1/9,9/1,0/1,0 ; Polyélectrolyte 8).

On charge dans un réacteur maintenu à 25°C sous agitation contenant 245 g de tertio butanol :
- 33,8g d'acide 2-acrylamido 2- méthyl propanesulfonique (ATBS) ;
- 12,9g d'hydrogénocarbonate d'ammonium ;
- 2,12g d'acrylate de (2-hydroxy éthyle) (HEA) ;
- 0,62g de méthacrylate de stéaryle (MAS) ;
- 0,48g de méthacrylate de lauryle (MALAU) ;
- 0,54g de triacrylate de triméthylol propane (TMPTA).

Après un temps suffisant pour atteindre une bonne homogénéisation de la solution, on désoxygène par barbotage d'azote puis on porte la température du milieu à 70°C. Lorsque la température désirée est atteinte, 0,50 g de peroxyde de dilauroyle sont ajoutés. La polymérisation démarre instantanément. On maintient alors le milieu réactionnel pendant environ 60 minutes à cette température, puis on chauffe à 80°C. Cette température est maintenue 2 heures. On ajoute alors du tertio-butylate de sodium de manière à échanger les ions ammonium par des ions sodium, puis on refroidit. La poudre qui s'est formée en cours de polymérisation est filtrée et séchée pour obtenir le polyélectrolyte 8.

### Exemple 9 (référence) : Polyélectrolyte réticulé de 2-méthyl 2-[(1-oxo 2-propènyl) amino] 1-propanesulfonate d'ammonium, d'acrylate de (2-hydroxy éthyle), de méthacylate d'isodécyle et de méthacrylate de lauryle, réticulé au triacrylate de triméthylolpropane (Polyélectrolyte 9).

On charge dans un réacteur maintenu à 25°C sous agitation contenant 245 g de tertio butanol :
- 33,8g d'acide 2-acrylamido 2-méthyl propanesulfonique (ATBS) ;
- 12,9g d'hydrogénocarbonate d'ammonium ;
- 2,12g d'acrylate de (2-hydroxy éthyle) (HEA) ;
- 0,41g de méthacrylate de d'isodécyle (MAID) ;
- 0,48g de méthacrylate de lauryle (MALAU) ;
- 0,54g de triacrylate de triméthylol propane (TMPTA).

Après un temps suffisant pour atteindre une bonne homogénéisation de la solution, on désoxygène par barbotage d'azote puis on porte la température du milieu à 70°C. Lorsque la température désirée est atteinte, 0,50 g de peroxyde de dilauroyle sont ajoutés. La polymérisation démarre instantanément. On maintient alors le milieu réactionnel pendant environ 60 minutes à cette température, puis on chauffe à 80°C. Cette température est maintenue 2 heures avant de refroidir. La poudre qui s'est formée en cours de polymérisation est filtrée et séchée pour obtenir le polyélectrolyte 9.

En procédant d'une manière similaire aux exemples précédents, les exemples suivants ont été préparés.

### Exemple 10 (selon l'invention) : Polyélectrolyte réticulé de 2-méthyl 2-[(1-oxo 2-propènyl)amino]1-propanesulfonate de sodium, d'acrylate de (2-hydroxy éthyle), de méthacylate de stéaryle et de méthacrylate de lauryle, réticulé au triacrylate de triméthylolpropane (ATBS/HEA/MAS/MALAU : 94/4/1/1 ; Polyélectrolyte 10).

### Exemple 11 (selon l'invention) : Polyélectrolyte réticulé de 2-méthyl 2-[(1-oxo 2-propènyl)amino]1-propanesulfonate de sodium, d'acrylate de (2-hydroxy éthyle), de méthacylate de stéaryle et de méthacrylate de lauryle, réticulé au triacrylate de triméthylolpropane (ATBS/HEA/MAS/MALAU : 83/15/1/1 ; Polyélectrolyte 11).

### Exemple 12 (selon l'invention) : Polyélectrolyte réticulé de 2-méthyl 2-[(1-oxo 2-propènyl) amino] 1-propanesulfonate de sodium, d'acrylate de (2-hydroxy éthyle), de méthacylate de stéaryle et de méthacrylate de lauryle, réticulé au triacrylate de triméthylolpropane (ATBS/HEA/MAS/MALAU : 88,2/9,8/1/1 ; Polyélectrolyte 12).

### Exemple 13 (selon l'invention) : Polyélectrolyte réticulé de 2-méthyl 2-[(1-oxo 2-propènyl) amino] 1-propanesulfonate de sodium, d'acrylate de (2-hydroxy éthyle), de méthacylate de stéaryle et de méthacrylate de lauryle, réticulé au triacrylate de triméthylolpropane (ATBS/HEA/MAS/MALAU : 88/9,6/1,2/1,2 ; Polyélectrolyte 13).

### Exemple 14 (selon l'invention) : Polyélectrolyte réticulé de 2-méthyl 2-[(1-oxo 2-propènyl) amino] 1-propanesulfonate de sodium, d'acrylate de (2-hydroxy éthyle), de méthacylate de stéaryle et de méthacrylate de lauryle, réticulé au triacrylate de triméthylolpropane (ATBS/HEA/MAS/MALAU : 82,5/14,5/1,5/1,5 ; Polyélectrolyte 14).

### Exemple 15 (selon l'invention) : Polyélectrolyte réticulé de 2-méthyl 2-[(1-oxo 2-propènyl) amino] 1-propanesulfonate de sodium, d'acrylate de (2-hydroxy éthyle), de méthacylate de stéaryle et de méthacrylate de lauryle, réticulé au triacrylate de triméthylolpropane (ATBS/HEA/MAS/MALAU : 89/10/0,5/0,5 ; Polyélectrolyte 15).

### B) Propriétés épaississantes :

Les épaississants selon l'invention ont été évalués comme suit :
**a)** - Mesure des viscosités (µ) :
   - à 2% massique dans l'eau,
   - et à 2% massique dans l'eau et comprenant 2% de chlorure de sodium.

Ces mesures de viscosité, exprimées en mPas, ont été effectuées à 25°C au moyen d'un rhéomètre Brookfield de type RVT équipé de l'axe numéro 6 et réglé à la vitesse de rotation de 5 tours par minute (rpm). Les mesures ont été effectuées juste après réalisation de la dispersion aqueuse. Les résultats sont consignés dans le tableau suivant :

| | Viscosité à 2% dans l'eau | Viscosité à 2% dans l'eau comprenant 2% de chlorure de sodium | Δµ= (µ₁-µ₀)/µ₀ |
|---|---|---|---|
| Polyélectrolyte 1 | µ₀= 55.800 mPas | µ₁= 32.400 mPas | -42% |
| Polyélectrolyte 2 | µ₀= 42.600 mPas | µ₁= 27.400 mPas | -36% |
| Polyélectrolyte 3 | µ₀= 45.400 mPas | µ₁= 31.800 mPas | -30% |
| Polyélectrolyte 4 | µ₀= 36.000 mPas | µ₁= 32.200 mPas | -11% |
| Polyélectrolyte 5 | µ₀= 60.000 mPas | µ₁= 15.200 mPas | -75% |
| Polyélectrolyte 6 | µ₀= 55.400 mPas | µ₁= 26.000 mPas | -53% |
| Polyélectrolyte 7 | µ₀= 24.600 mPas | µ₁= 21.600 mPas | -12% |
| Polyélectrolyte 8 | µ₀= 44.600 mPas | µ₁= 28.000 mPas | -37% |
| Polyélectrolyte 10 | µ₀= 40.600 mPas | µ₁= 37.000 mPas | -9% |
| Polyélectrolyte 11 | µ₀= 43.000 mPas | µ₁= 45.2000 mPas | +5% |
| Polyélectrolyte 12 | µ₀= 44.000 mPas | µ₁= 39.200 mPas | -11% |
| Polyélectrolyte 13 | µ₀= 44.800 mPas | µ₁= 35.800 mPas | -20% |
| Polyélectrolyte 14 | µ₀= 45.400 mPas | µ₁= 31.200 mPas | -31% |
| Polyélectrolyte 15 | µ₀= 41.400 mPas | µ₁= 29.200 mPas | -29% |

Ces résultats font apparaître que les polymères selon l'invention sont épaississants. Parmi ceux-ci les polymères 1, 2, 3, 4 et 8 à 15, sont plus particulièrement appropriés à une utilisation dans les formulations cosmétiques, leur dispersion aqueuse présentant une baisse de moins de 50% de leur viscosité en présence de sels tout ayant un caractère épaississant prononcé dans l'eau (> 30.000 mPas).

### C) Exemples de formulations préparées avec les polyélectrolytes selon l'invention (proportions exprimées en pourcentages massique)

### Exemple 1 : Crème de soin

| | |
|---|---|
| CYCLOMETHICONE™ : | 10% |
| Polyélectrolyte 1 : | 0,8% |
| MONTANOV™ 68 : | 2% |
| Alcool stéarylique : | 1% |
| Alcool stéarique : | 0,5% |
| Conservateur : | 0,65% |
| Lysine : | 0,025% |
| EDTA (sel disodique) : | 0,05% |
| Gomme xanthane : | 0,2% |
| Glycérine : | 3% |
| Eau : | q.s.p. 100% |

### Exemple 2 : Baume après-rasage

### FORMULE

| | | |
|---|---|---|
| A | Polyélectrolyte 2 : | 1,5% |
| | Eau : | q.s.p. 100% |
| B | MICROPEARL™ M 100 : | 5,0% |
| | SEPICIDE™ Cl : | 0,50% |
| | Parfum : | 0,20% |
| | Ethanol 95° : | 10,0% |

MODE OPERATOIRE : Ajouter B dans A.

### Exemple 3 : Emulsion satinée pour le corps

### FORMULE

| | | |
|---|---|---|
| A | SIMULSOL™ 165 : | 5,0% |
| | LANOL™ 1688 : | 8,50% |
| | Beurre de Karité : | 2% |
| | Huile de paraffine : | 6,5% |
| | LANOL™ 14M : | 3% |
| | LANOL™ S : | 0,6% |
| B | Eau : | 66,2% |
| C | MICROPEARL™ M 100 : | 5% |
| D | Polyélectrolyte 3 : | 3% |
| E | SEPICIDE™ Cl : | 0,3% |
| | SEPICIDE™ HB : | 0,5% |
| | AQUAXYL™ : | 3% |
| | Parfum : | 0,20% |
| | Acétate de vitamine E : | 0,20% |
| | Pyrolidinonecarboxylate de sodium : | 1% |

MODE OPERATOIRE : Ajouter C dans B, émulsionner B dans A à 70°C, puis ajouter D à 60°C puis E à 30°C.

### Exemple 4 : Crème H/E

### FORMULE

| | | |
|---|---|---|
| A | SIMULSOL™ 165 : | 5,0% |
| | LANOL™ 1688 : | 20,0% |
| | LANOL™ P : | 1,0% |
| B | Eau : | q.s.p. 100% |
| C | Polyélectrolyte 4 : | 2,50% |
| D | SEPICIDE™ Cl : | 0,20% |
| | SEPICIDE™ HB : | 0,30% |

MODE OPERATOIRE : Introduire B dans A vers 75°C ; ajouter C vers 60°C, puis D vers 45°C.

### Exemple 5 : Gel solaire non gras

### FORMULE

| | | |
|---|---|---|
| A | Polyélectrolyte 8 : | 3,00% |
| | Eau : | 30% |
| B | SEPICIDE™ Cl : | 0,20% |
| | SEPICIDE™ HB : | 0,30% |
| | Parfum : | 0,10% |
| C | Colorant : | q.s. |
| | Eau : | 30% |
| D | MICROPEARL™ M100 : | 3,00% |
| | Eau : | q.s.p. 100% |
| E | Huile de silicone : | 2,0% |
| | PARSOL™ MCX : | 5,00% |

MODE OPERATOIRE : Introduire B dans A ; ajouter C, puis D, puis E.

### Exemple 6 : Lait solaire

### FORMULE

| | | |
|---|---|---|
| A | MONTANOV™ S : | 3,0% |
| | Huile de sésame : | 5,0% |
| | PARSOL™ MCX : | 5,0% |
| | Carraghénane λ : | 0,10% |
| B | Eau : | q.s.p. 100% |
| C | Polyélectrolyte 3 : | 0,80% |
| D | Parfum : | q.s. |
| | Conservateur : | q.s. |

MODE OPERATOIRE : Emulsionner B dans A à 75°C puis ajouter C vers 60°C, puis D vers 30°C et ajuster le pH si nécessaire.

### Exemple 7 : Gel de massage

### FORMULE

| | | |
|---|---|---|
| A | Polyélectrolyte 2 : | 3,5% |
| | Eau : | 20,0% |
| B | Colorant: | 2 gouttes/100g |
| | Eau : | q.s. |
| C | Ethanol: | 10% |
| | Menthol : | 0,10% |
| D | Huile de silicone : | 5,0% |

MODE OPERATOIRE : Ajouter B dans A, puis ajouter au mélange, C puis D.

### Exemple 8 : Fond de teint hydratant et matifiant

### FORMULE

| | | |
|---|---|---|
| A | eau : | 20,0% |
| | Butylène glycol : | 4,0% |
| | PEG-400 : | 4,0% |
| | PECOSIL™ PS100 : | 1,0% |
| | Hydroxyde de sodium : | q.s. pH = 9 |
| | Dioxyde de titane : | 7,0% |
| | Talc : | 2,0% |
| | Oxyde de fer jaune : | 0,8% |
| | Oxyde de fer rouge : | 0,3% |
| | Oxyde de fer noir : | 0,05% |
| B | LANOL™ 99 : | 8% |
| | Caprylic capric triglycéride | 8% |
| | MONTANOV™ 202 : | 5,00% |
| C | eau : | q.s.p. 100% |
| | MICROPEARL™ M305 : | 2,0% |
| | EDTA tétrasodé : | 0,05% |
| D | CYCLOMETHICONE™ : | 4,0% |
| | Gomme xanthane : | 0,2% |
| | Polyélectrolyte 1 : | 0,8% |
| E | SEPICIDE™ HB : | 0,5% |
| | SEPICIDE Cl : | 03% |
| | Parfum : | 0,2% |

MODE OPERATOIRE : Préparer à 80°C, les mélanges B + D et A + C, puis mélanger et émulsionner l'ensemble.

### Exemple 9 : Gel coup d'éclat

### FORMULE

| | | |
|---|---|---|
| A | Polyélectrolyte 4 : | 4% |
| | Eau : | 30% |
| B | ELASTINE HPM™ : | 5,0% |
| C | MICROPEARL™ M100 : | 3% |
| | Eau : | 5% |
| D | SEPICIDE™ Cl : | 0,2% |
| | SEPICIDE™ HB : | 0,3% |
| | Parfum : | 0,06% |
| | Pyrolidinonecarboxylate de sodium 50% : | 1% |
| | Eau : | q. s. p. 100% |

MODE OPERATOIRE : Préparer A ; additionner B, puis C, puis D.

### Exemple 10 : Lait corporel

| | |
|---|---|
| MONTANOV™ S : | 3,5% |
| LANOL™ 37T : | 8,0% |
| SOLAGUM™ L : | 0,05% |
| Eau : | q.s.p.100% |
| Benzophénone-3 : | 2,0% |
| DIMETHICONE™ 350cPs : | 0,05% |
| Polyélectrolyte 3 : | 0,8% |
| Conservateur : | 0,2% |
| Parfum : | 0,4% |

### Exemple 11 : Emulsion démaquillante à l'huile d'amandes douces

| | |
|---|---|
| MONTANOV™ 68 : | 5% |
| Huile d'amandes douces : | 5% |
| Eau : | q.s.p.100% |
| Polyélectrolyte 8 : | 0,3% |
| Glycérine : | 5% |
| Conservateur : | 0,2% |
| Parfum : | 0,3% |

### Exemple 12 : Crème hydratante pour peaux grasses

| | |
|---|---|
| MONTANOV™ 68 : | 5% |
| Cétylstéaryloctanoate : | 8% |
| Palmitate d'octyle : | 2% |
| Eau : | q.s.p.100% |
| Polyélectrolyte 1 : | 0,6% |
| MICROPEARL™ M100 : | 3,0% |
| Mucopolysaccharides : | 5% |
| SEPICIDE™ HB : | 0,8% |
| Parfum : | 0,3% |

### Exemple 13 : Baume après-rasage apaisant sans alcool

| | |
|---|---|
| LIPACIDE™ PVB : | 1,0% |
| LANOL™ 99 : | 2,0% |
| Huile d'amandes douces : | 0,5% |
| Polyélectrolyte 4 : | 3,5% |
| Eau : | q.s.p.100% |
| Parfum : | 0,4% |
| SEPICIDE™ HB : | 0,4% |
| SEPICIDE™ Cl : | 0,2% |

### Exemple 14 : Crème aux AHA pour peaux sensibles

| | |
|---|---|
| Mélange de N-lauroyl aminoacides : | 0,1% à 5% |
| Aspartate de magnésium et de potassium : | 0,002% à 0,5% |
| LANOL™ 99 : | 2% |
| MONTANOV™ 68 : | 5,0% |
| Eau : | q.s.p.100% |
| Polyélectrolyte 2 : | 1,50% |
| Acide gluconique : | 1,50% |
| Triéthanolamine (TEA) : | 0,9% |
| SEPICIDE™ HB : | 0,3% |
| SEPICIDE™ Cl : | 0,2% |
| Parfum : | 0,4% |

### Exemple 15 : Soin apaisant après soleil

| | |
|---|---|
| Mélange de N-lauroyl aminoacides : | 0,1% à 5% |
| Aspartate de magnésium et de potassium : | 0,002% à 0,5% |
| LANOL™ 99 : | 10,0% |
| Eau : | q.s.p. 100% |
| Polyélectrolyte 3 : | 2,50% |
| SEPICIDE™ HB : | 0,3% |
| SEPICIDE™ Cl : | 0,2% |
| Parfum : | 0,4% |
| Colorant : | 0,03% |

### Exemple 16 : Lait démaquillant

| | |
|---|---|
| MONTANOV™ S : | 3% |
| PRIMOL™ 352 : | 8,0% |
| Huile d'amandes douces : | 2% |
| Eau : | q.s.p. 100% |
| Polyélectrolyte 2 : | 0,8% |
| Conservateur : | 0,2% |

### Exemple 17 : Emulsion fluide à pH alcalin

| | |
|---|---|
| MARCOL™ 82 : | 5,0% |
| Hydroxyde sodium : | 10,0% |
| Eau : | q.s.p. 100% |
| Polyélectrolyte 1 : | 1,5% |

### Exemple 18 : Fond de teint fluide

| | |
|---|---|
| SIMULSOL™ 165 : | 5,0% |
| LANOL™ 84D : | 8,0% |
| LANOL™ 99 : | 5,0% |
| Eau : | q.s.p. 100% |
| Pigments et charges minérales : | 10,0% |
| Polyélectrolyte 4 : | 1,2% |
| Conservateur : | 0,2% |
| Parfum : | 0,4% |

### Exemple 19 : Lait solaire

| | |
|---|---|
| MONTANOV™ S : | 3,5% |
| LANOL™ 37T : | 10,0% |
| PARSOL™ MCX : | 5,0% |
| EUSOLEX™ 4360 : | 2,0% |
| Eau : | q.s.p. 100% |
| Polyélectrolyte 8 : | 1,8% |
| Conservateur : | 0,2% |
| Parfum : | 0,4% |

### Exemple 20: Gel contour des veux

| | |
|---|---|
| Polyélectrolyte 1 : | 2,0% |
| Parfum : | 0,06% |
| Pyrolidinonecarboxylate de sodium : | 0,2% |
| DOW CORNlNG™ 245 Fluid : | 2,0% |
| Eau : | q.s.p. 100% |

### Exemple 21 : Composition de soin non rincée

| | |
|---|---|
| Polyélectrolyte 8 : | 1,5% |
| Parfum : | q.s. |
| Conservateur : | q. s. |
| DOW CORNING™ X2 8360 : | 5,0% |
| DOW CORNING™ Q2 1401 : | 15,0% |
| Eau : | q.s.p. 100% |

### Exemple 22: Gel amincissant

| | |
|---|---|
| Polyélectrolyte 4 : | 5% |
| Ethanol : | 30% |
| Menthol : | 0,1% |
| Caféine : | 2,5% |
| Extrait de ruscus : | 2% |
| Extrait de lierre : | 2% |
| SEPICIDE™ HB : | 1% |
| Eau : | q.s.p. 100% |

### Exemple 23 : Gel crème teinté ultra naturel

### FORMULE

| | | |
|---|---|---|
| A | Eau: | 10,0% |
| | Butylène glycol : | 4,0% |
| | PEG-400 : | 4,0% |
| | PECOSIL™ PS100 : | 1,5% |
| | Soude : | q.s. pH=7 |
| | Dioxyde de titane : | 2,0% |
| | Oxyde de fer jaune : | 0,8% |
| | Oxyde de fer rouge : | 0,3% |
| | Oxyde de fer noir : | 0,05% |
| B | LANOL™ 99 : | 4,0% |
| | Caprylic capric triglycéride | 4,0% |
| | SEPIFEEL™ ONE : | 1,0% |
| | Polyélectrolyte 3 : | 3,0% |
| C | Eau : | q.s.p. 100% |
| | MICROPEARL™ M305 : | 2,0% |
| | EDTA tétrasodé : | 0,05% |
| | CYCLOMETHYCONE™ : | 4,0% |
| D | SEPICIDE™ HB : | 0,5% |
| | SEPICIDE™ Cl : | 0,3% |
| | Parfum : | 0,2% |

| | | |
|---|---|---|
| | | |

MODE OPERATOIRE : Préparer le mélange B + C puis ajouter A puis D.

### Exemple 24 : Soin pour les peaux grasses

| | |
|---|---|
| MICROPEARL™ M310 : | 1,0% |
| Polyélectrolyte 2 : | 5,0% |
| Isononanoate d'octyle : | 4.0% |
| Eau : | q.s.p. 100% |
| SEPICONTROL™ A5 : | 4,0% |
| Parfum : | 0,1% |
| SEPICIDE™ HB : | 0,3% |
| SEPICIDE™ Cl : | 0,2% |
| CAPIGEL™ 98 : | 0,5% |
| Eau: | 10% |

### Exemple 25 : Crème aux AHA

| | |
|---|---|
| MONTANOV™ 68 : | 5,0% |
| LIPACIDE™ PVB: | 1,05% |
| LANOL™ 99 : | 10,0% |
| Eau : | q.s.p. 100% |
| Acide gluconique : | 1,5% |
| Triéthanolamine : | 0,9% |
| Polyélectrolyte 1 : | 1,5% |
| Parfum: | 0,4% |
| SEPICIDE™ HB: | 0,2% |
| SEPICIDE™ CI: | 0,4% |

### Exemple 26 : Autobronzant non gras pour visage et corps

| | |
|---|---|
| LANOL™ 2681 : | 3,0% |
| Polyélectrolyte 8 : | 2,5% |
| Eau : | q.s.p. 100% |
| Dihydroxyacétone : | 3,0% |
| Parfum : | 0,2% |
| SEPICIDE™ HB : | 0,8% |
| Soude : | q.s. pH = 5 |

### Exemple 27 : Lait solaire au monoï de Tahiti

| | |
|---|---|
| Monoï de Tahiti : | 10% |
| LIPACIDE™ PVB : | 0,5% |
| Polyélectrolyte 4 : | 2,2% |
| Eau : | q.s.p. 100% |
| Parfum : | 0,1% |
| SEPICIDE™ HB : | 0,3% |
| SEPICIDE™ CI : | 0,1% |
| PARSOL™ MCX : | 4,0% |

### Exemple 28 : Soin solaire pour le visage

| | |
|---|---|
| CYCLOMETHICONE™ et DIMETHICONOL™ : | 4,0% |
| Polyélectrolyte 3 : | 3,5% |
| Eau : | q.s.p. 100% |
| Parfum : | 0,1% |
| SEPICIDE™ HB : | 0,3% |

| | |
|---|---|
| SEPICIDE™ CI : | 0,21% |
| PARSOL™ MCX : | 5,0% |
| Micatitane : | 2,0% |
| Acide lactique : | q.s. pH = 6,5 |

### Exemple 29 : Emulsion bronzante sans soleil

| | |
|---|---|
| LANOL™ 99 : | 15% |
| MONTANOV™ 68 : | 5,0% |
| PARSOL™ MCX : | 3,0% |
| Eau : | q.s.p.100% |
| Dihydroxyacétone : | 5,0% |
| Phosphate monosodique : | 0,2% |
| Polyélectrolyte 2 : | 0,5% |
| Parfum : | 0,3% |
| SEPICIDE™ HB : | 0,8% |
| Soude : | q.s. pH=5. |

### Exemple 30 : Crème de soin

| | |
|---|---|
| CYCLOMETHICONE™ : | 10% |
| Polyélectrolyte 4 : | 0,8% |
| MONTANOV™ 68 : | 4,5% |
| Conservateur : | 0,65% |
| Lysine : | 0,025% |
| EDTA (sel disodique) : | 0,05% |
| Gomme xanthane : | 0,2% |
| Glycérine : | 3% |
| Eau : | q.s.p. 100% |

### Exemple 31 : Crème de soin

| | |
|---|---|
| CYCLOMETHYCONE™ : | 10% |
| Polyélectrolyte 3 : | 0,8% |
| MONTANOV™ 68 : | 4,5% |
| Perfluoropolyméthyl isopropyléther : | 0,5% |
| Conservateur : | 0,65% |
| Lysine : | 0,025% |
| EDTA (sel disodique) : | 0,05% |

| | |
|---|---|
| PEMULEN™ TR1 : | 0,2% |
| Glycérine : | 3% |
| Eau : | q.s.p. 100% |

### Exemple 32 : Lait corporel

### FORMULE

| | | |
|---|---|---|
| A | SIMULSOL™ 165 : | 5,0% |
| | LANOL™ 1688 : | 12,0% |
| | LANOL™ 14M : | 2,0% |
| | Alcool cétylique : | 0,3% |
| | SCHERCEMOL™ OP : | 3% |
| B | Eau : | q.s.p. 100% |
| C | Polyélectrolyte 2 : | 0,35% |
| D | SEPICIDE™ CI : | 0,2% |
| | SEPICIDE™ HB : | 0,5% |
| | Parfum : | 0,20% |

MODE OPERATOIRE : Emulsionner B dans A vers 75°C ; ajouter C vers 60°C, puis D vers 30°C;

### Exemple 33 : Gel soin de massage

### FORMULE

| | | |
|---|---|---|
| A | Polyélectrolyte 1 : | 3,00% |
| | Eau : | 30% |
| B | SEPICIDE™ CI : | 0,20% |
| | SEPICIDE™ HB : | 0,30% |
| | Parfum : | 0,05% |
| C | Colorant : | q.s. |
| | Eau : | q.s.p. 100% |
| D | MICROPEARL™ SQL : | 5,0% |
| | LANOL™ 1688 : | 2% |

MODE OPERATOIRE : Préparer A ; additionner B, puis C, puis D.

### Exemple 34 : Lait corporel

### FORMULE

| | | |
|---|---|---|
| A | MONTANOV™ S : | 3,0% |
| | Triheptonate de glycérol : | 10,0% |
| B | Eau : | q.s.p. 100% |
| C | Polyélectrolyte 8 : | 1,0% |
| D | Parfum : | q.s. |
| | Conservateur : | q.s. |

MODE OPERATOIRE : Fondre A à environ 75°C. Emulsionner B dans A à 75°C puis ajouter C vers 60°C, puis D.

### Exemple 35 : Baume après-rasage apaisant sans alcool

| | |
|---|---|
| Mélange de lauroyl aminoacides : | 0,1% à 5% |
| Aspartate de magnésium et de potassium : | 002% à 0,5% |
| LANOL™ 99 : | 2% |
| Huile d'amandes douces : | 0,5% |
| Eau : | q.s.p. 100% |
| Polyélectrolyte 2 : | 3% |
| SEPICIDE™ HB : | 0,3% |
| SEPICIDE™ CI : | 0,2% |
| Parfum : | 0,4% |

### Exemple 36 : Lait corporel

| | |
|---|---|
| MONTANOV™ S : | 3,5% |
| LANOL™ 37T : | 8,0% |
| SOLAGUM™ L : | 0,05% |
| Eau : | q.s.p. 100% |
| Benzophénone-3 : | 2,0% |
| DIMETHICONE™ 350 cPs : | 0,05% |
| Polyélectrolyte 4 : | 0,8% |
| Conservateur : | 0,2% |
| Parfum : | 0,4% |

### Exemple 37 : Baume après-rasage apaisant sans alcool

| | |
|---|---|
| LIPACIDE™ PVB : | 1,0% |
| LANOL™ 99 : | 2,0% |
| Huile d'amandes douces : | 0,5% |
| Polyélectrolyte 1 : | 3,5% |
| Eau : | q.s.p. 100% |
| Parfum : | 0,4% |

| | |
|---|---|
| SEPICIDE™ HB : | 0,4% |
| SEPICIDE™ CI : | 0,2% |

### Exemple 38 : Gel rafraîchissant après-rasage

| | |
|---|---|
| LIPACIDE™ PVB : | 0,5% |
| LANOL™ 99 : | 5,0% |
| Polyélectrolyte 1 : | 2,5% |
| Eau : | q.s.p. 100% |
| MICROPEARL™ LM : | 0,5% |
| Parfum : | 0,2% |
| SEPICIDE™ HB : | 0,3% |
| SEPICIDE™ CI : | 0,2% |

### Exemple 39 : Crème aux AHA

| | |
|---|---|
| MONTANOV™ 68 : | 5,0% |
| LIPACIDE™ PVB : | 1,05% |
| LANOL™ 99 : | 10,0% |
| Eau : | q.s.p. 100% |
| Acide gluconique : | 1,5% |
| Triéthanolamine : | 0,9% |
| Polyélectrolyte 4 : | 1,5% |
| Parfum : | 0,4% |
| SEPICIDE™ HB : | 0,2% |
| SEPICIDE™ CI : | 0,4% |

### Exemple 40 : Gel brillance

| | |
|---|---|
| Polyélectrolyte 2 : | 1,5% |
| Silicone volatile : | 25% |
| Monopropylèneglycol : | 25% |
| Eau déminéralisée : | 10% |
| Glycérine : | q.s.p. 100% |

### Exemple 41 : Gel amincissant

| | |
|---|---|
| Polyélectrolyte 3 : | 1,5% |
| Isononanoate d'isononyle : | 2% |
| Caféine : | 5% |

| | |
|---|---|
| Ethanol : | 40% |
| MICROPEARL™ LM : | 2% |
| Eau déminéralisée : | q.s.p. 100% |
| Conservateur parfum : | q.s. |

### Exemple 42 : Lait démaquillant

| | |
|---|---|
| SIMULSOL™ 165 : | 4% |
| MONTANOV™ 202 : | 1% |
| Caprylate-caprate triglycéride : | 15% |
| PECOSIL™ DCT : | 1% |
| Eau déminéralisée : | q.s. |
| CAPIGEL™ 98 : | 0,5% |
| Polyélectrolyte 15 : | 1% |
| PROTEOL™ APL: | 2% |
| Hydroxyde de sodium : | q.s.p. pH = 7 |

### Exemple 43 : Masque crème "rince off" restructurant pour cheveux stressés et fragilisés

| | |
|---|---|
| KETROL™T : | 0,5% |
| PECOSIL™ SPP50 : | 0,75% |
| N-cocoyl aminoacides : | 0,70% |
| Butylèneglycol : | 3,0% |
| Polyélectrolyte 4 : | 3,0% |
| MONTANOV™ 82 : | 3,0% |
| Huile de jojoba : | 1,0% |
| LANOL™ P : | 6,0% |
| AMONYL™ DM : | 1,0% |
| LANOL™ 99 : | 5,0% |
| SEPICIDE™ HB : | 0,3% |
| SEPICIDE™CI : | 0,2% |
| Parfum : | 0,2% |
| Eau : | q.s.p. 100% |

### Exemple 44 : Crème solaire

| | |
|---|---|
| SIMULSOL™ 165 : | 3% |
| MONTANOV™ 202 : | 2% |

| | |
|---|---|
| Benzoate C12-C15 : | 8% |
| PECOSIL™ PS 100 : | 2% |
| DIMETHICONE™ : | 2% |
| CYCLOMETHICONE™ : | 5% |
| Para-méthoxy cinnamate d'octyle : | 6% |
| Benzophénone-3 : | 4% |
| Oxyde de Titane : | 8% |
| Gomme xanthane : | 0,2% |
| Butylèneglycol : | 5% |
| Eau déminéralisée : | q.s.p. 100% |
| Polyélectrolyte 1 : | 1,5% |
| Conservateur, parfum : | q.s. |

### Exemple 45 : Gel de soin peaux mixtes

| | |
|---|---|
| Polyélectrolyte 4 : | 4% |
| Squalane végétal : | 5% |
| DIMETHYCONE : | 1,5% |
| SEPICONTROL™ A5 : | 4% |
| Gomme xanthane : | 0,3% |
| Eau : | q.s.p. 100% |
| Conservateur, Parfum : | q.s. |

### Exemple 46 : Lotion capillaire

| | |
|---|---|
| Butylène glycol : | 3,0% |
| Polyélectrolyte 2 : | 3% |
| SIMULSOL™1293 : | 3,0% |
| Acide lactique : | q.s. pH = 6 |
| SEPICIDE™ HB : | 0,2% |
| SEPICIDE™CI : | 0,3% |
| Parfum : | 0,3% |
| Eau : | q.s.100% |

### Exemple 47 : Shampooing protecteur et relaxant

| | |
|---|---|
| AMONYL™ 675 SB : | 5,0% |
| Sodium lauroyl éther sulfate à 28% : | 35,0% |
| Polyélectrolyte 4 : | 3,0% |

| | |
|---|---|
| SEPICIDE™ HB: | 0,5% |
| SEPICIDE™CI : | 0,3% |
| Hydroxyde de sodium : | q.s. pH = 7,2 |
| Parfum : | 0,3% |
| Colorant (FDC bleu 1/jaune 5) : | q.s. |
| Eau : | q.s.p. 100% |

### Exemple 48 : Protecteur "leave-on" ; Soin anti-stress pour cheveux

| | |
|---|---|
| KETROL™T : | 0,5% |
| Mélange de cocoyl aminoacides : | 3,0% |
| Butylèneglycol : | 5,0% |
| DC 1501 : | 5,0% |
| Polyélectrolyte 4 : | 4,0% |
| SEPICIDE™ HB: | 0,5% |
| SEPICIDE™CI : | 0,3% |
| Parfum : | 0,3% |
| Eau : | q.s.p. 100% |

### Exemple 49 : Crème vitaminée

| | |
|---|---|
| SIMULSOL™ 165 : | 5% |
| MONTANOV™ 202 : | 1% |
| Caprylic/capric triglycérides : | 20% |
| Palmitate de vitamine A : | 0,2% |
| Acétate de vitamine E : | 1% |
| MICROPEARL™ M305 : | 1,5% |
| Polyélectrolyte 3 : | 2% |
| Eau | q.s.p. 100% |
| Conservateur, parfum | q.s. |

### Exemple 50 : Gel solaire

| | |
|---|---|
| Polyélectrolyte 8 : | 3,00% |
| SEPICIDE™ CI : | 0,20% |
| SEPICIDE™ HB : | 0,30% |
| Parfum : | 0,10% |
| Colorant : | q.s. |
| Silice : | 3,00% |

| | |
|---|---|
| Eau : | q.s.p. 100% |
| Huile de silicone : | 2,0% |
| Benzophénone-3 : | 5,00% |

### Exemple 51 : Gloss lèvre

| | |
|---|---|
| Polyélectrolyte 3 : | 1,50% |
| SCHERCEMOL™ TISC : | 15,00% |
| VISTANOL™ NPGC : | 15,00% |
| CANDURIN PAPRIKA™ : | 0,50% |
| MONTANOX™ 80 : | 1,00% |
| ANTARON™ V216 : | 0,90% |
| Arôme Abricot : | 0,20% |
| SEPICIDE™ HB : | 0,50% |
| C MALTIDEX™ H16322 : | q.s.p. 100% |

### Exemple 52 : Poudre pressée Terre de soleil

| | |
|---|---|
| Polyélectrolyte 1 : | 2,00% |
| LANOL™ 99 : | 12,00% |
| SEPIWHITE™ MSH : | 1,00% |
| Talc : | 33,00% |
| MICROPEARL™ M310 : | 3,00% |
| Oxyde de fer jaune : | 0,80% |
| Oxyde de fer rouge : | 0,30% |
| Oxyde de fer noir : | 0,05% |
| Mica : | q.s. 100% |

### Exemple 53 : Emulsion pour peaux à tendance atopique

| | |
|---|---|
| ARLACEL™ P135 : | 2,00% |
| Polyélectrolyte 4 : | 1,00% |
| LANOL™ 1688 : | 14,00% |
| PRIMOL™ 352 : | 8,00% |
| Glycérine : | 5,00% |
| Eau : | q.s.p. 100% |
| Sulfate de magnésium : | 0,70% |
| SEPICIDE™ HB : | 0,30% |
| SEPICIDE™ CI : | 0,20% |

| | |
|---|---|
| MICROPEARL™ M310 : | 5,00% |

### Exemple 54 : Soin solaire apaisant (eau dans silicone)

| | |
|---|---|
| Polyélectrolyte 2 : | 2,00% |
| DC5225C : | 20,00% |
| DC345 : | 10,00% |
| SEPICALM™ VG : | 3,00% |
| Dioxide de titane MT100T : | 5,00% |
| Oxyde de zinc Z cote HP1 : | 5,00% |
| SEPICIDE™ HB : | 0,30% |
| Parfum : | 0,05% |
| SEPICIDE™ CI : | 0,20% |
| Glycérine : | 5,00% |
| Chlorure de sodium : | 2,00% |
| Eau : | q.s.p. 100% |

### Exemple 55 : Soin multi-phases

| | |
|---|---|
| Polyélectrolyte 4 : | 3,00% |
| C12-15 alkylbenzoate : | 25,00% |
| AQUAXYL™ : | 3,00% |
| SEPITONIC™ M3 : | 1,00% |
| SEPICIDE™ HB : | 0,50% |
| SEPICIDE™ CI : | 0,30% |

### Exemple 56 : Gel autobronzant

| | |
|---|---|
| Polyélectrolyte 3 : | 5,0% |
| Ethanol : | 30% |
| Dihydroxyacétone : | 5% |
| Menthol : | 0,1% |
| Caféine : | 2,5% |
| Extrait d'Ivry : | 2% |
| SEPICIDE™ HB : | 1% |
| Eau : | q.s.p. 100% |

### Exemple 57 : Gel solaire et autobronzant

| | |
|---|---|
| MONTANOV™ S : | 3,0% |

| | |
|---|---|
| Triheptanoate de glycéryle : | 10,0% |
| LIPACIDE™ PVB : | 1,05% |
| Polyélectrolyte 2 : | 2,2% |
| Eau : | q.s. 100% |
| Dihydroxyacétone : | 5% |
| Parfum : | 0,1% |
| SEPICIDE™ HB : | 0,3% |
| SEPICIDE™ CI : | 0,1% |
| PARSOL™ MCX : | 4,0% |

### Exemple 58 : Crème autobronzante aux α-hydroxy acides

| | |
|---|---|
| MONTANOV™ 68 : | 5,0% |
| LIPACIDE™ PVB : | 1,05% |
| LANOL™ 99 : | 10,0% |
| Eau : | q.s. 100% |
| Acide gluconique : | 1,5% |
| Dihydroxyacétone : | 3% |
| Triéthanolamine : | 0,9% |
| Polyélectrolyte 1 : | 1,5% |
| Parfum : | 0,4% |
| SEPICIDE™ HB : | 0,2% |
| SEPICIDE™ CI : | 0,4% |

### Exemple 59 : Crème autobronzante aux a-hydroxy acides pour peau sensible

| | |
|---|---|
| Mélange de N-lauroyl aminoacides : | 0,1% à 5% |
| Aspartate de magnésium et de potassium : | 0,002% à 0,5% |
| MONTANOV™ 68 : | 5,0% |
| LANOL™ 99 : | 2,0% |
| Eau : | q.s. 100% |
| Acide lactique : | 1,5% |
| Dihydroxyacétone : | 3,5% |
| Triéthanolamine : | 0,9% |
| Polyélectrolyte 2 : | 1,5% |
| Parfum : | 0,4% |
| SEPICIDE™ HB : | 0,3% |
| SEPICIDE™ CI : | 0,2% |

### Exemple 60 : Emulsion hydratante auto-bronzante satinée

| | |
|---|---|
| SIMULSOL™ 165 : | 5,0% |
| LANOL™ 1688 : | 8,5% |
| Beurre de Galam : | 2% |
| Paraffine liquide : | 6,5% |
| LANOL™ 14M : | 3% |
| LANOL™ S : | 0,6% |
| Eau : | 66,2% |
| Dihydroxyacétone : | 3% |
| MICROPEARL™ M100 : | 5% |
| Polyélectrolyte 3 : | 3% |
| AQUAXYL™ : | 5% |
| Acétate de vitamine E : | 0,20% |
| Pyrolidinonecarboxylate de sodium : | 0,20% |
| Parfum : | 0,2% |
| SEPICIDE™ HB : | 0,5% |
| SEPICIDE™ CI : | 0,3% |

### Exemple 61 : Spray solaire organo-minéral

### FORMULE

| | | |
|---|---|---|
| A | Néopentanoate d'isodécyle : | 20% |
| | CYCLODIMETHICONE™ : | 5% |
| | Méthoxycinnamate d'éthyl hexyle : | 6% |
| | Butyl méthoxy dibenzoylméthane : | 3% |
| | DL alpha-Tocophérol : | 0,05% |
| B | Eau : | q.s.p. 100% |
| | EDTA tétrasodique : | 0,2% |
| | Glycérine : | 7% |
| | Acide phényl Benzimidazolesulfonique (salifié avec quantité molaire nécessaire de soude) : | 3% |
| C | Polyélectrolyte 8 : | 1,3% |
| | KETROL™CG-T : | 0,315% |
| | EFFICACIA™M : | 0,385% |
| D | SEPICIDE™ HB: | 1% |
| | Fragrance : | 0,1% |

### Exemple 62 : Crème corporelle

### FORMULE:

| | |
|---|---|
| Triglycérides 4555 (C8C10) : | 12% |
| C12-C15 alkyl Benzoate : | 5,3% |
| Isohexadécane: | 2,7% |
| Alcool cétylique : | 2% |
| DL alpha-Tocophérol : | 0,10% |
| Polyélectrolyte 2 : | 1,5% |
| Gomme de Tara : | 0,5% |
| Eau : | q.s.p. 100% |
| GIVOBIO™ GZn : | 1% |
| SEPICALM™ S : | 3% |
| EUXYL™ PE910 : | 1% |
| Fragrance : | 0,1% |

### Exemple 63 : gel parfumant dynamisant

| | | |
|---|---|---|
| A | Polyélectrolyte 3 : | 2,25% |
| | Aqua/Water: | q.s.p. 100% |
| B | Huile essentielle de citron : | 0,50% |
| | Huile essentielle de sauge : | 0,50% |
| | Glycérine : | 2,00% |
| | Ethanol : | 83,00% |

### Exemple 64 : Gel capillaire parfumé protecteur UV

| | | |
|---|---|---|
| A | Polyélectrolyte 4 : | 1,00% |
| | SOLAGUM™ AX : | 1,00% |
| | Disodium EDTA : | 0,05% |
| | Aqua/Water: | q.s.p. 100% |
| | Colorant : | q.s. |
| B | Disodium Phenyl Benzimidazole Tetrasulfonate : | 5,00% |
| | Benzophénone-4 : | 3,00% |
| | Aminométhyl Propanol : | 4,00% |
| | Aqua/Water : | 25,00% |
| C | Concentré de parfum A : | 0,10% |
| | Phénoxyéthanol et Ethylhexyl Glycérine : | 1,00% |

| | | |
|---|---|---|
| | Polysorbate 20 : | 0,50% |
| | PEG/PPG-20/15 DIMETHYCONE™ : | 1,00% |
| | Ethanol : | 20,00% |
| D | Triéthanolamine : | q.s. pH=7-7.2 |

### Exemple 65 : Eau de Cologne gélifiée

| | | |
|---|---|---|
| A | Polyélectrolyte 10 : | 3,00% |
| | Aqua/Water: | q.s.p. 100% |
| B | Concentré de parfum B : | 4,00% |
| | Ethanol : | 81,00% |

### Exemple 66 : gel coiffant

| | |
|---|---|
| Polyélectrolyte 4 : | 2,00% |
| Polyvinylpyrrolidone : | 2,00% |
| VP/Méthacrylamide/n-Vinyl Imidazole Copolymer | 15% |
| Ethanol : | 20% |
| Polysorbate 20 : | 2,5% |
| Fragrance : | 0,3% |
| DIMETHYCONE COPOLYOL™ : | 1,0% |
| Eau : | q.s.p. 100% |

### Exemple 67 : Emulsion eau-dans-huile parfumante

| | | |
|---|---|---|
| A | Isononate d'isononyle : | 6% |
| | Ethylhexyl glycérine : | 1% |
| B | EASYNOV™ : | 2,5% |
| C | Polyélectrolyte 12 : | 0,5% |
| | AQUAXYL™ : | 3% |
| | Eau : | q.s.p. 100% |
| D | Ethanol: | 40% |
| | Parfum : | 2,0% |

### Exemple 68 : Emulsion eau-dans-huile anti-transpirante

| | | |
|---|---|---|
| A | Isononate d'isononyle : | 6% |
| | Ethylhexyl glycérine : | 1% |
| B | EASYNOV™ : | 2,5% |
| C | Polyélectrolyte 8 : | 0,5% |
| | AQUAXYL™ : | 3% |
| | Eau : | q.s.p. 100% |
| | Chlorhydrate d'aluminium (50%) : | 30% |

### Exemple 69: Emulsion eau-dans-huile éclaircissante

| | | |
|---|---|---|
| A | Isononate d'isononyle : | 6% |
| | Fragrance : | 0,1% |
| | Phénoxy éthanol & éthylhexyl glycérine : | 1% |
| B | EASYNOV™ : | 1,5% |
| | Polyélectrolyte 2 : | 0,5% |
| | SEPIWHITE™MSH : | 2% |
| | Eau : | q.s.p. 100% |

### Exemple 70 : Gel douche sans sulfate

| | |
|---|---|
| AMONYL™ 380 BA (28,9% M.A.) : | 10,4% |
| ORAMIX™NS10 ⁽(54,2% MA) : | 22,73% |
| Eau : | q.s.p. 100% |
| Acide lactique en solution à 45% : | 0,3% |
| Polyélectrolyte 1 : | 2% |

### Exemple 71 : Gel douche

| | |
|---|---|
| AMONYL™ 380 BA (28,9% M.A.) : | 10,4% |
| Lauryl éther (2,2 OE) sulfate de sodium (27,1% M.A) | 44,3% |
| Eau | q.s.p. 100% |
| Acide lactique en solution à 45% | 0,3% |
| Polyélectrolyte 3 | 0,8% |

### Exemple 72 : Shampooing

| | | |
|---|---|---|
| A | JAGUAR™ C14 S : | 0,20% |
| | Eau : | q.s.100% |
| | Polyélectrolyte 2 : | 0,20% |
| B | EDTA disodique : | 0,05% |
| C | Sodium Lauryl Ether Sulfate 70% : | 16,0% |
| | AMONYL™ 380 BA : | 3,0% |
| | ORAMIX™ NS10 : | 4,0% |
| | Glycol Distéarate : | 1,5% |
| | Acool cétéarylique | 0,5% |
| | Coconut Ethanol Amide : | 0,8% |
| | Coconut Diethanol Amide : | 0,8% |
| D | Polyquaternium-7 : | 2,0% |

| | | |
|---|---|---|
| | Dimethicone & Laureth-23 & Laureth-3 : | 1,0% |
| | Dimethiconol & TEA-Dodecyl benzenesulfonate : | 2,0% |
| | SEPICIDE™ HB : | 1,0% |
| | Fragrance : | 0,3% |
| | Chlorure de sodium | 0,8% |
| | Acide citrique (25%) : | qs pH = 6,0 |

### Exemple 73 : shampooing 2 en 1

| | | |
|---|---|---|
| A | JAGUAR™C14 S : | 0,2% |
| | Eau : | qsp 100% |
| | Polyélectrolyte 1 : | 0,2% |
| B | EDTA disodique : | 0,05% |
| C | Sodium Lauryl Ether Sulfate 70% : | 16,0% |
| | AMONYL™ 380 BA : | 3,0% |
| | ORAMIX™ NS10 | 4,0% |
| | Glycol distéarate : | 1,5% |
| | Alcool cétéarylique | 0,5% |
| | Coconut Ethanol Amide | 0, 8% |
| | Coconut Diethanol Amide | 0, 8% |
| D | Polyquaternium-7 | 1, 8% |
| | Polyquaternium-10 | 0, 2% |
| | Dimethicone & Laureth-23 & Laureth-3 | 1, 0% |
| | Dimethiconol & TEA-Dodecyl benzenesulfonate | 2, 0% |
| | SEPICIDE™ HB : | 1,0% |
| | Fragrance : | 0,3% |
| | Chlorure de sodium : | 0,8% |
| | Acide citrique (25%) : | q.s. pH = 6,0 |

### Exemple 74 : spray démêlant anti-frisottis

| | | |
|---|---|---|
| A | MONTANOV™ L : | 0,5% |
| | Cyclopentasiloxane and Dimethiconol : | 2,0% |
| B | Polyélectrolyte 2 : | 1,2% |
| | Eau : | Qsp 60% |
| C | Eau : | Qsp 100% |
| | Chlorure de Cetrimonium : | 0,5% |
| D | SEPICIDE™ HB: | 1,0% |

| | | |
|---|---|---|
| | Parfum/Fragrance : | 0,1% |
| | Acide lactique: | q.s. pH = 6,0 |

Les produits commerciaux utilisés dans les exemples, sont définis comme suit :
- SIMULSOL™ 1293 est de l'huile de castor hydrogénée et éthoxylée, avec un indice d'éthoxylation égal à 40, commercialisé par la société SEPPIC ;
- CAPIGEL™ 98 est un épaississant liquide à base de copolymère acrylate commercialisé pas la société SEPPIC ;
- KELTROL™CG-T et KETROL™ T sont la gomme xanthane commercialisé par la société CP KELCO ;
- LANOL™ 99 est de l'isononanoate d'isononyle commercialisé par la société SEPPIC ;
- DC 1501™ est un mélange de cyclopentasiloxane et de diméthiconol commercialisé par la société DOW CHEMICAL ;
- MONTANOV™ 82 est un agent émulsionnant à base d'alcool cétéarylique et de cocoylglucoside ;
- MONTANOV™ 68 (cétéaryl glucoside), est une composition auto-émulsionnable telle que décrite dans WO 92/06778, commercialisée par la société SEPPIC ;
- MICROPEARL™ M100 est une poudre ultra fine au toucher très doux et à action matifiante commercialisée par la société MATSUMO ;
- SEPICIDE™ CI, imidazolidine urée, est un agent conservateur commercialisé par la société SEPPIC ;
- PEMULEN™ TR1 est un polymère acrylique commercialisé par GOODRICH ;
- SIMULSOL™ 165 est du stéarate de glycérol auto-émulsionnable commercialisée par la société SEPPIC ;
- LANOL™ 1688 est un ester émollient à effet non gras commercialisé par la société SEPPIC ;
- LANOL™ 14M et LANOL™ S sont des facteurs de consistance commercialisés par la société SEPPIC ;
- SEPICIDE™ HB (nom INCI : Phenoxyethanol / Methylparaben / Ethylparaben / Propylparaben / Butylparaben) est un agent conservateur commercialisé par la société SEPPIC ;
- AQUAXYL™ (nom INCI : Xylitylglucoside and Anhydroxylitol and Xylitol) : Composition hydratante commercialisée par la société SEPPIC ;
- SCHERCEMOL™ OP est un ester émollient à effet non gras ;
- LANOL™ P est un additif à effet stabilisant commercialisé par la société SEPPIC ;
- PARSOL™ MCX est du para-méthoxy cinnamate d'octyle ; commercialisé par la société GIVAUDAN ;
- MONTANOV™ S est un agent nacrant, commercialisé par la société SEPPIC, à base d'un mélange d'alkyl poly glucosides tels que ceux décrits dans WO 95/13863 ;
- MICROPEARL™ SQL est un mélange de micro particules renfermant du squalane qui se libère sous l'action du massage; il est commercialisé par la société MATSUMO ;
- LANOL™ 37T est du triheptanoate de glycérol, commercialisé par la société SEPPIC ;
- SOLAGUM™ L est un carraghénane commercialisé par la société SEPPIC ;
- MARCOL™ 82 est une huile de paraffine commercialisée par la société EXXON ;
- LANOL™ 84D est du malate de dioctyle commercialisé par la société SEPPIC ;
- PARSOL™ NOX est un filtre solaire commercialisé par la société GIVAUDAN ;
- EUSOLEX™ 4360 est un filtre solaire commercialisé par la société MERCK ;
- DOW CORNING™ 245 (ou DC 245™) Fluid est de la CYCLOMETHYCONE, commercialisée par la société DOW CORNING ;
- LIPACIDE™ PVB, est un hydrolysât de protéines de blé acylé commercialisé par la société SEPPIC ;
- MICROPEARL™ LM est un mélange de squalane, de polyméthylméthacrylate et de menthol, commercialisé par la société SEPPIC ;
- SEPICONTROL™ A5 est un mélange de capryloy glycine, de sarcosine, d'extrait de cannelle (cinnamon zylanicum), commercialisé par la société SEPPIC, tel que ceux décrits dans la demande internationale de brevet PCT/FR98/01313 déposée le 23 juin 1998 ;
- LANOL™ 2681 est un mélange caprylate, caprate de coprah, commercialisé par la société SEPPIC ;
- MONTANOV™ 202, est une composition APG/alcools gras telle que décrite dans WO 98/47610, commercialisée par la société SEPPIC ;
- PROTEOL™ APL est un tensioactif moussant, commercialisée par la société SEPPIC ;
- SCHERCEMOL™ TISC est un ester (citrate de tri-isostéaryle) commercialisé par la société SCHER ;
- VISTANOL™ NPGC est un ester (néopentyl glycol dicaprate) commercialisé par la société SEWA KASEI ;
- ANTARON™ V216 est un polymère synthétique (PVP/hexadecene copolymer) distribué par la société UNIVAR ;
- C MALTIDEX™ H16322 est un polyol (sirop de maltitol) commercialisé par la société CERESTAR;
- SEPIWHITE™MSH (nom INCI: ω-undecylenoyl phenylalanine) est un agent éclaircissant de la peau commercialisé par la société SEPPIC ;
- DC 345™ est un CYCLOMETHYCONE commercialisé par la société DOW CORNING ;
- DC 5225C™ est un mélange de cyclopentasiloxane et de DIMETHYCONE copolyol commercialisé par la société DOW CORNING ;
- SEPICALM™ VG est un actif apaisant (sodium palmitoylproline) commercialisé par la société SEPPIC ;
- MT100VT est un dioxyde de titane micronisé ayant subi un traitement de surface (hydroxyde d'aluminium/acide stéarique) distribué par la société UNIPEX ;
- Z COTE HP1™ est un oxyde de zinc micronisé ayant subi un traitement de surface distribué par GATTEFOSSE.
- CANDURIN PAPRIKA™ est un mélange de silicate de potassium et d'aluminium et d'oxyde de fer ;
- MICROPEARL™ M310 est une poudre ultra fine au toucher très doux et à action matifiante commercialisée par la société MATSUMO ;
- PRIMOL™ 352 est une huile minérale commercialisée par la société EXXON ;
- PECOSIL™DCT est du sodium DIMETHYCONE PEG-7 Acetyl Methyltaurate commercialisé par la société PHOENIX ;
- PECOSIL™PS 100 est du DIMETHYCONE PEG-7 commercialisé par la société PHOENIX ;
- EFFICACIA™M est la gomme d'acacia commercialisé par la société CNI ;
- EUXYL™ PE910 est un mélange de Phénoxyéthanol et d'Ethylhexylglycérine commercialisé par la société Schülke & Mayr ;
- GIVOBlO™ GZn (nom INCI : Zinc Gluconate) : Composition commercialisée par la société SEPPIC ;
- SEPICALM™ S : (nom INCI : Sodium Cocoyl Aminoacids and Sarcosine and Potassium Aspartate and Magnésium Aspartate) : Composition anti-inflammatoire commercialisée par la société SEPPIC ;
- SOLAGUM™ AX (Acacia Senegal Gum and Xanthan Gum) est un polymère épaississant et stabilisant commercialisé par la société SEPPIC ;
- EASYNOV™ (nom INCI : Octyldodecanol, Octyldodecyl Xyloside and PEG-30 Dipolyhydroxystearate) est une composition émulsionnante commercialisée par la société SEPPIC ;
- Concentré de parfum A : Fragrance 61303658 commercialisé par la société Drom ;
- Concentré de parfum B : « Eau verte », commercialisé par la société MLW ;
- AMONYL™ 380 BA est une cocamidopropyl betaïne, commercialisée par la société SEPPIC ;
- ORAMIX™NS 10 est une composition moussante comprenant du décylpolyglucoside, du dodécylpolyglucoside et du tétradécylpolyglucoside, commercialisée par la société SEPPIC ;
- JAGUAR" C14 S (nom INCI : Guar Hydroxypropyltrimonium Chloride) est un agent conditionnant commercialisé par la société Rhodia ;
- Polyquaternium-7 est un polymère synthétique cationique, se présentant sous la forme d'un dérivé ammonium quaternaire, et utilisé comme agent filmogène et comme agent antistatique ;
- Polyquaternium-10 est un polymère d'hydroxyéthyl cellulose se présentant sous la forme d'un dérivé quaternisé, et utilisé comme agent épaississant ;
- MONTANOV™ L est un agent émulsionnant commercialisé par la société SEPPIC ;
- SEPICIDE™ HB (nom INCI: Phenoxyethanol /Methylparaben /Ethylparaben/ Propylparaben Butyl paraben) est un agent conservateur.

## Revendications

1. Polyélectrolyte anionique réticulé issu de la polymérisation pour 100% molaire :
(i) - D'une proportion molaire supérieure ou égale à 75% molaire et inférieure ou égale à 95% molaire d'unités monomériques issues de l'acide 2-méthyl 2-[(1-oxo 2-pro-pènyl) amino] 1-propanesulfonique partiellement salifié ou totalement salifié sous forme de sel de sodium ou de sel d'ammonium ;
(ii) - D'une proportion supérieure ou égale à 0,5% molaire inférieure ou égale à 5% molaire d'unités monomériques issues du méthacrylate de lauryle de formule (I₁), et du méthacrylate de stéaryle de formule (I₂), dans un ratio molaire. (I₁)/(I₂) supérieur ou égal à 1/6 et inférieur ou égal à 6/1 ; avec la formule (I₁), correspondant à la formule (I) :
CH₂=CH(CH₃)-C(=O)-OR₁ (I)
dans laquelle R₁ représente un radical dodécyle et la formule (I₂), correspondant à la formule (I) dans laquelle R₁ représente un radical octadécyle ;
(iii) - D'une proportion supérieure ou égale à 0,5% molaire et inférieure ou égale à 3,0% molaire d'unités monomériques issues du triméthylol propanetriacrylate;
(iv) - Et d'une proportion molaire supérieure ou égale à 4,0% molaire et inférieure ou égale à 20% molaire d'unités monomériques issues d'acrylate de (2-hydroxy éthyle).

2. Polyélectrolyte anionique réticulé selon la revendication 1, issu de la polymérisation pour 100% molaire:
(i) - D'une proportion supérieure ou égale à 83% molaire et inférieure ou égale à 90% molaire d'unités monomériques issues de l'acide 2-méthyl 2-[(1-oxo 2-pro-pènyl) amino] 1-propanesulfonique partiellement salifié ou totalement salifié sous forme de sel de sodium ou de sel d'ammonium ;
(ii) - D'une proportion supérieure ou égale à 1,5% molaire et inférieure ou égale à 2,5% d'unités monomériques issues du méthacrylate de lauryle de formule (I₁), et du méthacrylate de stéaryle de formule (I₂), dans un ratio molaire. (I₁)/(I₂) supérieur ou égal à 1/6 et inférieur ou égal à 6/1 ;
(iii) - D'une proportion supérieure ou égale à 0,5% molaire et inférieure ou égale à 3,0% molaire d'unités monomériques issues du triméthylol propanetriacrylate ;
(iv) - D'une proportion supérieure ou égale à 8% molaire à inférieure ou égale à 15% molaires d'unités monomériques issues d'acrylate de (2-hydroxy éthyle).

3. Procédé de préparation du polyélectrolyte tel que défini à l'une quelconque des revendications 1 ou 2, **caractérisé en ce qu'**il comprend :
- Une étape a) au cours de laquelle on mélange l'ensemble des monomères, un agent de neutralisation ammoniaqué du monomère possédant une fonction acide fort, l'agent réticulant et si nécessaire ou si désiré, les autres monomères et/ou additifs dans un solvant organique, de préférence le tert-butanol ;
- Une étape b) au cours de laquelle la réaction de polymérisation est amorcée par introduction d'un initiateur de radicaux libres dans la dispersion préparée à l'étape a), puis est laissée se dérouler jusqu'à sa complétude ;
- Optionnellement une étape c) d'échange de l'ion ammonium présent par l'ion sodium ou l'ion potassium ;
- Une étape d) de précipitation du polymère formé à l'issue de l'étape b), optionnellement de l'étape c) ;
- Optionnellement une étape e) de séchage du précipité obtenu à l'issue de l'étape d).

4. Utilisation du polyélectrolyte anionique tel que défini à l'une quelconque des revendications 1 ou 2, comme épaississant et/ou comme stabilisant et/ou comme émulsionnant, d'une composition topique cosmétique, dermopharmaceutique ou pharmaceutique.

5. Composition topique, comprenant de 0,1% et 10% massique du polyélectrolyte anionique tel que défini à l'une quelconque des revendications 1 ou 2.

## Patentansprüche

1. Aus der Polymerisation stammender vernetzter anionischer Polyelektrolyt, für 100 Mol-%:
(i) - aus einem molaren Anteil größer als oder gleich 75 Mol-% und kleiner als oder gleich 95 Mol-% an Monomereinheiten, stammend von, in der Form des Natriumsalzes oder Ammoniumsalzes, teilweise in ein Salz überführter oder vollständig in ein Salz überführter 2-Methyl-2-[(1-oxo-2-propenyl)amino]1-propansulfonsäure;
(ii) - aus einem Anteil größer als oder gleich 0,5 Mol-%, kleiner als oder gleich 5 Mol-% an Monomereinheiten, stammend von Laurylmethacrylat von Formel (I₁) und von Stearylmethacrylat von Formel (I₂), in einem molaren Verhältnis. (I₁)/(I₂) größer als oder gleich 1/6 und kleiner als oder gleich 6/1; wobei die Formel (I₁) der Formel (I) entspricht:
CH₂=CH(CH₃)-C(=O)-OR₁ (I)
worin R₁ einen Dodecylrest und die Formel (I₂) darstellt, entsprechend der Formel (I), worin R₁ einen Octadecylrest darstellt;
(iii) - aus einem Anteil größer als oder gleich 0,5 Mol-% und kleiner als oder gleich 3,0 Mol-% an von Trimethylolpropantriacrylat stammenden Monomereinheiten;
(iv) - und aus einem molaren Anteil größer als oder gleich 4,0 Mol-% und kleiner als oder gleich 20 Mol-% an von (2-Hydroxyethylacrylat) stammenden Monomereinheiten.

2. Aus der Polymerisation stammender vernetzter anionischer Polyelektrolyt nach Anspruch 1, für 100 Mol-%:
(i) - aus einem Anteil größer als oder gleich 83 Mol-% und kleiner als oder gleich 90 Mol-% an Monomereinheiten, stammend von, in der Form des Natriumsalzes oder Ammoniumsalzes, teilweise in ein Salz überführter oder vollständig in ein Salz überführter 2-Methyl-2-[(1-oxo-2-propenyl)amino]-1-propansulfonsäure;
(ii) - aus einem Anteil größer als oder gleich 1,5 Mol-% und kleiner als oder gleich 2,5 % an Monomereinheiten, stammend von Laurylmethacrylat von Formel (I₁) und von Stearylmethacrylat von Formel (I₂), in einem molaren Verhältnis. (I₁)/(I₂) größer als oder gleich 1/6 und kleiner als oder gleich 6/1;
(iii) - aus einem Anteil größer als oder gleich 0,5 Mol-% und kleiner als oder gleich 3,0 Mol-% an von Trimethylolpropantriacrylat stammenden Monomereinheiten;
(iv) - einem Anteil größer als oder gleich 8 Mol-% bis kleiner als oder gleich 15 Mol-% an von (2-Hydroxyethylacrylat) stammenden Monomereinheiten.

3. Polyelektrolyt-Zubereitungsverfahren nach einem der Ansprüche 1 oder 2, **dadurch gekennzeichnet, dass** es umfasst:
- einen Schritt a), in dessen Verlauf die Gesamtheit an Monomeren, ein ammoniakhaltiges Neutralisierungsmittel des Monomers, das eine starke Säurefunktion besitzt, das Vernetzungsmittel und, wenn notwendig oder wenn gewünscht, die anderen Monomere und/oder Zusatzstoffe in einem organischen Lösemittel, vorzugsweise tert-Butanol, gemischt werden;
- einen Schritt b), in dessen Verlauf die Polymerisationsreaktion durch Einführung eines Initiators freier Radikale in die in Schritt a) zubereitete Dispersion initiiert wird, anschließend bis zu ihrer Vollständigkeit laufen gelassen wird;
- wahlweise einen Schritt c) des Austauschs des vorhandenen Ammoniumions durch das Natriumion oder das Kaliumion;
- einen Schritt d) des Ausfällens des am Ende von Schritt b), wahlweise von Schritt c), gebildeten Polymers;
- wahlweise einen Schritt e) des Trocknens des am Ende von Schritt d) erhaltenen Niederschlags.

4. Verwendung des anionischen Polyelektrolyten nach einem der Ansprüche 1 oder 2, als Verdickungsmittel und/oder als Stabilisator und/oder als Emulgator einer topischen kosmetischen, dermopharmazeutischen oder pharmazeutischen Zusammensetzung.

5. Topische Zusammensetzung, umfassend zu 0,1 Massen-% und 10 Massen-% anionischen Polyelektrolyten nach einem der Ansprüche 1 oder 2.

## Claims

1. Cross-linked anionic polyelectrolyte resulting from the polymerization for 100 mol%:
(i) - Of a molar ratio greater than or equal to 75 mol% and less than or equal to 95 mol% of monomeric units resulting from partially or totally salified 2-methyl-2-[(1-oxo-2-propenyl)amino]-1-propanesulfonic acid in the form of sodium salt or ammonia salt;
(ii) - Of a ratio greater than or equal to 0.5 mol% less than or equal to 5 mol% of monomeric units resulting from lauryl methacrylate having formula (I₁), and stearyl methacrylate having formula (I₂), in a molar ratio (I₁)/(I₂) greater than or equal to 1/6 and less than or equal to 6/1; with the formula (I₁), corresponding to the formula (I):
CH₂=CH(CH₃)-C(=O)-OR₁ (1)
wherein R₁ is a dodecyl radical and the formula (I₂), corresponding to the formula (I) wherein R₁ is an octadecyl radical;
(iii) - Of a ratio greater than or equal to 0.5 mol% and less than or equal to 3.0 mol% of monomeric units resulting from trimethylolpropane triacrylate;
(iv) - And of a molar ratio greater than or equal to 4.0 mol% and less than or equal to 20 mol% of monomeric units resulting from 2-hydroxyethyl acrylate.

2. Cross-linked anionic polyelectrolyte according to claim 1, resulting from the polymerization for 100 mol%:
(i) - Of a ratio greater than or equal to 83 mol% and less than or equal to 90 mol% of monomeric units resulting from partially or totally salified 2-methyl-2-[(1-oxo-2-propenyl)amino]-1-propanesulfonic acid in the form of sodium salt or ammonia salt;
(ii) - Of a ratio greater than or equal to 1.5 mol% and less than or equal to 2.5% of monomeric units resulting from lauryl methacrylate having formula (I₁), and stearyl methacrylate having formula (I₂), in a molar ratio (I₁)/(I₂) greater than or equal to 1/6 and less than or equal to 6/1;
(iii) - Of a ratio greater than or equal to 0.5 mol% and less than or equal to 3.0 mol% of monomeric units resulting from trimethylolpropane triacrylate;
(iv) - Of a ratio greater than or equal to 8 mol% and less than or equal to 15 mol% of monomeric units resulting from 2-hydroxyethyl acrylate.

3. Method for preparing polyelectrolyte such as defined in any of claims 1 or 2, **characterised in that** it comprises:
- A step a) during which are mixed all of the monomers, an ammonia neutralisation agent of the monomer having a strong acid function, the cross-linking agent and if necessary or if desired, the other monomers and/or additives in an organic solvent, more preferably tert-butanol;
- A step b) during which the polymerisation reaction is triggered by the introduction of a free radical initiator into the dispersion prepared in the step a), and is then allowed to continue until it is completed;
- Optionally a step c) of exchanging the ammonium ion present with the sodium ion or the potassium ion;
- A step d) of precipitating the polymer formed as a result of the step b), optionally of the step c);
- Optionally a step e) of drying the precipitate obtained at the end of the step d).

4. Use of the anionic polyelectrolyte such as defined in any of claims 1 or 2, as a thickener and/or as a stabiliser and/or as an emulsifier, of a cosmetic, dermopharmaceutical or pharmaceutical topical composition.

5. Topical composition, comprising from 0.1 mol% to 10 mol% of anionic polyelectrolyte such as defined in any of claims 1 or 2.
